# EUROPEAN PATENT SPECIFICATION

(11) **EP 2 792 696 B1**
(45) Date of publication and mention of the grant of the patent: **21.03.2018**
(21) Application number: 14168544.6
(22) Date of filing: 23.10.2008
(51) Int. Cl.: C08G 63/08, C08L 67/04, A61L 27/18, A61L 31/06, A61L 31/10

(54) **BIODEGRADABLE POLYMERIC MATERIALS PROVIDING CONTROLLED RELEASE OF HYDROPHOBIC DRUGS FROM IMPLANTABLE DEVICES**
Biodegradierbare Polymermaterialien mit kontrollierter Freisetzung hydrophober Arzneistoffe aus implantierbaren Vorrichtungen
Matériaux polymères biodégradables assurant une libération contrôlée de médicaments hydrophobes à partir de dispositifs implantables

(30) Priority: 31.10.2007 US 982160
(43) Date of publication of application: 22.10.2014
(62) Divisional of application: 08845756.9
(73) Proprietor: Abbott Cardiovascular Systems Inc., Santa Clara, CA 95054-2807 (US)
(72) Inventor: Lim, Florencia, Union City, CA California 94587 (US); Ngo, Michael, Huy, San Jose, CA California 95131 (US); Tang, Yiwen, Sunnyvale, CA California 94086 (US); Hossainy, Syed, F. A., Hayward, CA California 94544 (US); Trollsas, Mikael, O., San Jose, CA California 95124 (US)
(74) Representative: ZBM Patents - Zea, Barlocci & Markvardsen

(56) References cited:
- EP-A- 0 420 541
- EP-A- 0 761 712
- WO-A-03/020330
- WO-A-2004/108111
- US-A1- 2003 139 567

## Description

### BACKGROUND

### Field of the Invention

The present invention is directed to stents formed of biodegradable polymeric materials providing controlled release of hydrophobic drugs.

### Description of the State of the Art

Angioplasty is a well-known procedure for treating heart disease. A problem associated with angioplasty includes formation of intimal flaps or torn arterial linings which can collapse and occlude the conduit after the balloon is deflated. Moreover, thrombosis and restenosis of the artery may develop over several months after angioplasty, which may require another angioplasty procedure or a surgical by-pass operation. "Stenosis" refers to a narrowing or constriction of the diameter of a bodily passage or orifice, and "restenosis" refers to the reoccurrence of stenosis in a blood vessel or heart valve after it has been treated (as by balloon angioplasty, stenting, or valvuloplasty) with apparent success.

Stents are often used in the treatment of atherosclerotic stenoses in blood vessels. To reduce the partial or total occlusion of the artery by the collapse of arterial lining and to reduce the chance of thrombosis and restenosis following angioplasty in the vascular system, a stent can be implanted in the lumen to reinforce body vessels and maintain the vascular patency. A "lumen" refers to a cavity of a tubular organ such as a blood vessel. As a mechanical intervention, stents act as scaffoldings, functioning to physically hold open and, if desired, to expand the wall of a passageway, e.g., a blood vessel, urinary tract or bile duct.

Stents are also used as a vehicle for providing biological therapy. Biological therapy can be achieved by medicating the stents. Medicated stents provide for the local administration of a therapeutic substance at the treatment site, thereby possibly avoiding side effects associated with systemic administration of such substance. One method of medicating stents involves the use of a polymeric carrier coated over the surface of a stent, wherein a therapeutic substance is impregnated in the polymeric carrier.

Late stent thrombosis has emerged as a concern for drug-delivery stents. The incidence of late stent thrombosis appears to be higher with drug-delivery stents than with the corresponding bare metal stents. One potential cause of late thrombosis with drug-delivery stents is a chronic inflammatory or hypersensitivity response to the polymeric coating over the stent.

To reduce the risk of late stent thrombosis, the stent can be coated with a biodegradable polymer derived from one or more hydrophilic monomers. At typical drug-to-polymer mass ratios, however, many hydrophobic drugs exhibit unsatisfactory release profiles with conventional biodegradable polymers. By contrast, the present invention provides biodegradable polymeric materials that release hydrophobic drugs from implantable devices in a controlled manner.

The document EP0420541 (SQUIBB BRISTOL MYERS CO) discloses the preparation of ureteral stents and infusion stents (a type of ureteral stents) from biodegradable polymers of lactic acid. The polymer composition preferably comprises a terpolymer of L(-)lactide, glycolide and epsilon-caprolactone and the speed of resorption of the stent is controlled by varying the molecular weight. This document does not disclose any peripheral or coronary stent, or using the stent for drug delivery.

### SUMMARY OF THE INVENTION

The present invention is directed to stents formed of compositions comprising biodegradable polymeric materials that provide controlled release of hydrophobic drugs from said stents, as defined in the claims. The polymeric materials are biodegradable copolymers derived from three or more monomers, wherein at least two monomers are relatively polar so as to completely or substantially completely erode after the devices accomplish their intended functions (e.g., maintaining vascular patency and locally delivering drugs), thereby avoiding adverse effects such as late stent thrombosis. Further, at least one monomer is relatively nonpolar monomer that increases the miscibility of hydrophobic drugs with the polymer and enhance the polymer's permeability to hydrophobic drugs. Other advantages of the biodegradable polymeric materials forming the stent include, e.g., good mechanical properties (e.g., toughness and flexibility) and good physical properties (e.g., degradation rate and drug-release rate).

The invention relates to stents formed of a composition comprising a biodegradable copolymer,
the biodegradable copolymer being derived from three or more monomers;
wherein the biodegradable copolymer is derived from:
at least two monomers selected from glycolide (GA), D-lactide (DLA), L-lactide (LLA), D,L-lactide (DLLA), and meso-lactide (MLA); and
at least one monomer selected from valerolactone (VL), caprolactone (CL), trimethylene carbonate (TMC), dioxanone (DS), hydroxybutyrate (HB), and hydroxyvalerate (HV);
wherein the stent is a coronary stent or a peripheral stent;
wherein the biodegradable copolymer has a Tg from -150 °C to 100 C;
wherein the biodegradable copolymer has a polymer number-average molecular weight (Mn) from 10 kDa to 500 kDa;
wherein the biodegradable copolymer completely degrades, which is the loss of at least 95 % of its mass, substantially completely degrades, which is the loss of at least 75% of its mass, within 12 months, when exposed to physiological conditions;
   and
wherein for the biodegradable copolymer each kind of monomer independently has from 10 to 5,000 units in the copolymer; and the molar % of each kind of monomer independently is from 5% to 90%.

In one embodiment, the at least two monomers are selected from, D-lactide (DLA), L-lactide (LLA), D,L-lactide (DLLA) and meso-lactide (MLA), and the at least one monomer is selected from valerolactone (VL), caprolactone (CL), trimethylene carbonate (TMC), dioxanone (DS), hydroxybutyrate (HB) and hydroxyvalerate (HV). In one embodiment, the biodegradable copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

In some embodiments, the biodegradable copolymer has a degree of crystallinity of less than 50% or less than 20%. In certain embodiments, the copolymer is amorphous.

In further embodiments, the composition additionally comprises one or more components selected from biocompatible moieties, non-fouling moieties, biobeneficial materials, and biologically active agents, where the bioactive agents have a sustained release up to 12 months. In certain embodiments, the bioactive agents are hydrophobic drugs, e.g., rapamycin and derivatives thereof.

Further embodiments of the invention are directed to stents as defined above for use in the treatment or prevention of a condition or disorder in a patient, selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, and arteriovenous anastamoses,

Various embodiments of the invention are described in further detail below.

### BRIEF DESCRIPTION OF THE DRAWINGS

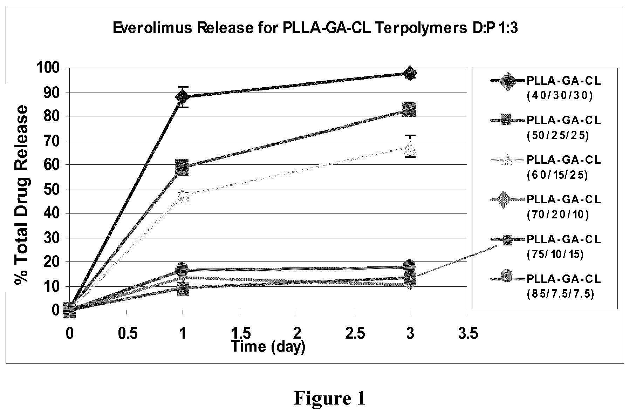

**Figure 1** shows the release rates of everolimus from stents coated with P(LLA-GA-CL) terpolymers containing various molar percentages of LLA, GA and CL, where the D:P ratio was 1:3 and the dosage of everolimus was about 100 microgram/cm².

### DETAILED DESCRIPTION OF THE INVENTION

### Terms and Definitions

The following definitions apply:

The terms "biologically degradable" (or "biodegradable"), "biologically erodable" (or "bioerodable"), "biologically absorbable" (or "bioabsorbable"), and "biologically resorbable" (or "bioresorbable"), in reference to polymers and coatings, are used interchangeably and refer to polymers and coatings that are capable of being completely or substantially completely degraded, dissolved, and/or eroded over time when exposed to physiological conditions and can be gradually resorbed, absorbed and/or eliminated by the body, or that can be degraded into fragments that can pass through the kidney membrane of an animal (e.g., a human), e.g., fragments having a molecular weight of about 40,000 Daltons (40 kDa) or less. The process of breaking down and eventual absorption and elimination of the polymer or coating can be caused by, e.g., hydrolysis, metabolic processes, oxidation, enzymatic processes, bulk or surface erosion, and the like. Conversely, a "biostable" polymer or coating refers to a polymer or coating that is not biodegradable.

Whenever reference is made to "biologically degradable," "biologically erodable," "biologically absorbable," or "biologically resorbable" stent coatings or polymers forming such stent coatings, it is understood that after the process of degradation, erosion, absorption, and/or resorption has been completed or substantially completed, no coating or substantially little coating will remain on the stent.

"Complete degradation" of a polymer or a polymeric material (e.g., a polymeric coating) means that the polymer or the polymeric material loses at least 95% of its mass over a period of time.

"Substantially complete degradation" of a polymer or a polymeric material (e.g., a polymeric coating) means that the polymer or the polymeric material loses at least 75% of its mass over a period of time. In certain embodiments, "substantially complete degradation" of a polymer or a polymeric material can mean that the polymer or the polymeric material loses at least 80% of its mass, or at least 85% of its mass, or at least 90% of its mass, or at least 95% of its mass over a period of time.

As used herein, a "biocompatible moiety" refers to a moiety that is capable of enhancing the biological compatibility of the composition, material (e.g., coating) or structure (e.g., implantable device) containing it by not causing injury or toxicity to, or an immunological reaction in, living tissue.

A "biobeneficial material" refers to a material that benefits a treatment site (e.g., by enhancing the biocompatibility of the implantable device containing such material) by being, e.g., non-fouling, hemocompatible, non-thrombogenic, and/or anti-inflammatory without depending on the release of a pharmaceutically or therapeutically active agent.

A "non-fouling moiety" is a moiety that provides an implantable device fabricated from or coated with a material comprising the moiety with the ability to resist (i.e., to prevent, delay, or reduce the amount of) build-up of a denatured layer of protein on its surface, which is caused by the body's reaction to foreign material and could lead to protein fouling. The adsorption of proteins on the surface of an implanted device constitutes the first step of several biological responses, including the activation of the coagulation cascade. Following protein adsorption, cell adhesion occurs, which could lead to impairment of the device's functioning as well as adverse side effects on the patient. For example, thrombi formation could occur after adsorption and activation of platelets.

"Physiological conditions" refer to conditions to which an implant is exposed within the body of an animal (e.g., a human). Physiological conditions include, but are not limited to, "normal" body temperature for that species of animal (approximately 37 °C for a human) and an aqueous environment of physiologic ionic strength, pH and enzymes. In some cases, the body temperature of a particular animal may be above or below what would be considered "normal" body temperature for that species of animal. For example, the body temperature of a human may be above or below approximately 37 °C in certain cases. The scope of the present invention encompasses those cases where the physiological conditions (e.g., body temperature) of an animal are not considered "normal".

In the context of a blood-contacting implantable device, a "prohealing" drug or agent refers to a drug or agent that has the property that it promotes or enhances re-endothelialization of arterial lumen to promote healing of the vascular tissue.

As used herein, a "co-drug" is a drug that is administered concurrently or sequentially with another drug to achieve a particular pharmacological effect. The effect may be general or specific. The co-drug may exert an effect different from that of the other drug, or it may promote, enhance or potentiate the effect of the other drug.

As used herein, the term "prodrug" refers to an agent rendered less active by a chemical or biological moiety, which metabolizes into or undergoes *in vivo* hydrolysis to form a drug or an active ingredient thereof. The term "prodrug" can be used interchangeably with terms such as "proagent", "latentiated drugs", "bioreversible derivatives", and "congeners". N.J. Harper, Drug latentiation, Prog Drug Res., 4: 221-294 (1962); E.B. Roche, Design of Biopharmaceutical Properties through Prodrugs and Analogs, Washington, DC: American Pharmaceutical Association (1977); A.A. Sinkula and S.H. Yalkowsky, Rationale for design of biologically reversible drug derivatives: prodrugs, J. Pharm. Sci., 64: 181-210 (1975). Use of the term "prodrug" usually implies a covalent link between a drug and a chemical moiety, though some authors also use it to characterize some forms of salts of the active drug molecule. Although there is no strict universal definition of a prodrug itself, and the definition may vary from author to author, prodrugs can generally be defined as pharmacologically inactive or less active chemical derivatives that can be converted *in vivo*, enzymatically or nonenzymatically, to the active, or more active, drug molecules that exert a therapeutic, prophylactic or diagnostic effect. Sinkula and Yalkowsky, above; V.J. Stella et al., Prodrugs: Do they have advantages in clinical practice?, Drugs, 29: 455-473 (1985).

The terms "polymer" and "polymeric" refer to compounds that are the product of a polymerization reaction. These terms are inclusive of homopolymers (i.e., polymers obtained by polymerizing one kind of monomer), copolymers (i.e., polymers obtained by polymerizing two or more different kinds of monomers), terpolymers (i.e., polymers obtained by polymerizing three different kinds of monomers), etc., including random, alternating, block, graft, star, dendritic, crosslinked and any other variations thereof.

The terms "block copolymer" and "graft copolymer" are defined in accordance with the terminology used by the International Union of Pure and Applied Chemistry (IUPAC). "Block copolymer" refers to a copolymer containing a linear arrangement of blocks. The block is defined as a portion of a polymer molecule in which the monomer units have at least one constitutional or configurational feature absent from the adjacent portions. "Graft copolymer" refers to a polymer composed of macromolecules with one or more species of block connected to the main chain as side chains, these side chains having constitutional or configurational features that differ from those in the main chain.

As used herein, a "polar monomer" is a monomer that is more polar than a "nonpolar monomer". Likewise, a "nonpolar monomer" is a monomer that is less polar than a "polar monomer". The terms "polar monomer" and "relatively polar monomer" are used interchangeably herein, and the terms "nonpolar monomer" and "relatively nonpolar monomer" are also used interchangeably herein. In some embodiments, a "polar monomer" is a monomer that is more hydrophilic than a "nonpolar monomer". Likewise, in some embodiments a "nonpolar monomer" is a monomer that is more hydrophobic than a "polar monomer".

A stent can be designed for the localized delivery of a therapeutic agent. A medicated stent can be constructed in part, e.g., by coating the device with a coating material containing a therapeutic agent. The body of the device can also contain a therapeutic agent.

A stent can be coated with a coating containing partially or completely a biodegradable/bioabsorbable/bioerodable polymer, a biostable polymer, or a combination thereof. A portion of the stent or the whole device itself can also be fabricated partially or completely from a biodegradable/bioabsorbable/bioerodable polymer, a biostable polymer, or a combination thereof.

As used herein, a "portion" of a stent can be any portion of the stent. For example, a portion can be a portion of the body of the stent. As another example, a portion can be a portion of the surface of the stent, or the whole surface of the stent. As a further example, a portion can refer to an area of material in the body or over the surface of the stent, e.g., a layer, film or coating disposed over the stent.

As used herein, a material (e.g., a layer, film or coating) "disposed over" a substrate (e.g., an implantable device) is deposited directly or indirectly over at least a portion of the surface of the substrate. Direct depositing means that the material is applied directly to the exposed surface of the substrate. Indirect depositing means that the material is applied to an intervening material that has been deposited directly or indirectly over the substrate.

The "glass transition temperature", Tg, is the temperature at which the amorphous domains of a polymer change from a solid glassy state to a solid deformable or rubbery state at atmospheric pressure. In other words, the T_{g} corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised, the actual molecular volume in the sample remains constant, and so a higher coefficient of expansion points to an increase in free volume associated with the system and therefore increased freedom for the molecules to move. The increasing heat capacity corresponds to an increase in heat dissipation through movement. The T_{g} of a given polymer can be dependent on the heating rate and can be influenced by the thermal history of the polymer. Furthermore, the chemical structure of the polymer heavily influences the glass transition by affecting chain mobility.

An "aliphatic" group is an optionally substituted, straight-chain or branched, saturated or unsaturated hydrocarbon moiety. If unsaturated, the aliphatic group may contain one or more double bonds and/or one or more triple bonds. The aliphatic group is divalent (i.e., -R-) in terms of its attachment to the rest of the compound.

A "heteroaliphatic" group is an aliphatic group that contains at least one heteroatom selected from O, S and N, in the main portion and/or the branch(es) of the hydrocarbon moiety.

A "cycloaliphatic" group is an optionally substituted, saturated or unsaturated, mono- or polycyclic hydrocarbon moiety. If unsaturated, the cycloaliphatic group may contain one or more double bonds in and/or off of one or more rings of the cyclic moiety. The cycloaliphatic group is divalent (i.e., -Cyc-) in terms of its attachment to the rest of the compound, but one or both of the points of attachment may be directly on one or more rings of the cyclic moiety (e.g., -cyclohexyl-) or via one or more groups attached to the ring(s) (e.g., -CH₂-cyclohexyl-CH₂-).

A "heterocycloaliphatic" group is a cycloaliphatic group in which at least one ring in the cyclic moiety contains one or more heteroatoms selected from O, S, and N.

An "aromatic" group is an optionally substituted mono- or polycyclic aromatic moiety. The ring(s) in the moiety may be aromatic or non-aromatic (saturated or unsaturated), but at least one ring in the moiety is aromatic. The aromatic ring(s) in the moiety are carbocyclic, but any non-aromatic ring in the moiety may contain one or more heteroatoms selected from O, S, and N. The aromatic group is divalent (i.e., -Ar-) in terms of its attachment to the rest of the compound, but one or both of the points of attachment may be directly on one or more aromatic or non-aromatic rings of the cyclic moiety (e.g.,-phenyl-) or may be via one or more groups attached to the ring(s) (e.g., -CH₂-phenyl-CH₂-).

A "heteroaromatic" group is an aromatic group in which at least one aromatic ring in the aromatic moiety contains one or more heteroatoms selected from O, S, and N.

The aliphatic, heteroaliphatic, cycloaliphatic, heterocycloaliphatic, aromatic and heteroaromatic groups may be substituted or unsubstituted. If substituted, they may contain from 1 to 5 substituents. The substituents include, but are not limited to: optionally substituted carbon-containing groups, e.g., alkyl, cycloalkyl and aryl; halogen atoms (i.e., F, Cl, Br and I) and optionally substituted halogen-containing groups (e.g., haloalkyl); optionally substituted oxygen-containing groups, e.g., oxo, alcohols and ethers; optionally substituted carbonyl-containing groups, e.g., aldehydes, ketones, carboxy acids, esters, carbonates, thioesters, amides, carbamates and ureas; optionally substituted groups containing carbonyl derivatives, e.g., imines, oximes and thioureas; optionally substituted nitrogen-containing groups, e.g., amines, azides, nitriles and nitro; optionally substituted sulfur-containing groups, e.g., thiols, sulfides, thioethers, sulfoxides, sulfones, sulfonates and sulfonamides; and optionally substituted aromatic or non-aromatic heterocyclic groups containing one or more heteroatoms selected from O, S and N.

### Embodiments of stent of the Invention

### Composition and Copolymer

To minimize or prevent adverse side effects associated with a polymer, a drug-delivery stent can be coated with a biodegradable polymer. Examples of such polymers can be derived from highly degradable monomers such as lactide and glycolide. For example, a poly(D,L-lactide-co-glycolide) (PDLGA) copolymer containing 75% D,L-lactide (DLLA) and 25% glycolide (GA) substantially completely degrades within about six months. However, PDLGA copolymers provide unsatisfactory controlled release of hydrophobic drugs such as rapamycin and derivatives thereof (e.g., everolimus). Due to this deficiency, PDLGA copolymers have a narrow process and formulation window with respect to the mass ratio of hydrophobic drug to polymer (D:P). For example, at a D:P mass ratio of 1:1, the PDLGA 75/25 copolymer has a tendency to release everolimus in a burst (> 95% drug release within 24 hours). At D:P ratios of 1:3 and 1:5, PDLGA 75/25 releases everolimus at a substantially lower rate (< 15% drug release over a period of three days). Only at very specific process and formulation conditions does PDLGA 75/25 provide a controlled release of drugs such as everolimus.

In contrast to PDLGA copolymers, the biodegradable copolymers forming the stents of the invention provide controlled release of hydrophobic drugs. The inventive copolymers are derived from at least two relatively polar monomers as defined in claim 1 and at least one relatively nonpolar monomer as defined in claim 1. The at least two relatively polar monomers impart a higher T_{g} and biodegradability to the copolymer. In some embodiments, the relatively polar monomers are relatively hydrophilic. The at least one relatively nonpolar monomer, being relatively hydrophobic, increases the miscibility of a hydrophobic drug with the copolymer. For example, the at least one relatively nonpolar monomer provides a relatively hydrophobic moiety for better phase mix of the copolymer with the hydrophobic drug. The increased miscibility of the hydrophobic drug with the copolymer improves the controlled release of the drug from a polymeric implantable device at lower drug-to-polymer mass ratios (i.e., increasing amounts of polymer relative to drug).

In addition, the at least one relatively nonpolar monomer imparts good mechanical properties (e.g., fracture toughness and flexibility) and good physical properties (e.g., drug permeability) to the copolymer. The mechanical and physical properties of the copolymer can be tuned by appropriate selection of the relatively polar and nonpolar monomers and the ratio and arrangement thereof. For example, the at least one relatively nonpolar monomer and its amount can be selected so as to form a more amorphous copolymer having a lower T_{g}, which would increase the diffusivity of a hydrophobic drug through the copolymer. Further, to improve the biological properties of a stent formed of a material comprising the copolymer, one or more biocompatible moieties, non-fouling moieties, and/or biobeneficial materials can be physically or chemically attached to, blended with, or incorporated with the copolymer.

According to the invention, the stent is formed of a composition comprising a biodegradable copolymer, wherein the copolymer:
is derived from at least two polar monomers as defined in claim 1 and at least one nonpolar monomer as defined in claim 1;
has a T_{g} from -150 °C to 100 °C;
has a polymer number-average molecular weight (Mₙ) from 10 kDa to 500 kDa; and
completely or substantially completely degrades within 12 months. When exposed to physiological conditions;
and wherein:
each kind of monomer independently has from 10 to 5,000 units in the copolymer; and
the molar % of each kind of monomer independently is from 5% to 90%.

In a narrower embodiment, optionally in combination with one or more other embodiments described herein, the copolymer:
has a T_{g} from -100 °C to 100 °C; and
has an Mₙ from 20 kDa to 500 kDa;
and:
each kind of monomer independently has from 50 to 3,000 units in the copolymer;
the molar % of each of the at least two polar monomers independently is from 5% to 85%; and
the molar % of each of the at least one nonpolar monomer independently is from 5% to 50%.

In one embodiment, the copolymer has a T_{g} within a specified range when it is hydrated. In another embodiment, the copolymer has T_{g} within a specified range when it is not hydrated.

The copolymer of the invention has any T_{g} value within the range from -150 °C to 100 °C. The T_{g} of the copolymer can be tuned to a desired value depending on the particular applications of the copolymer. In narrower embodiments, the copolymer can have a T_{g} from -125 °C to 100 °C, or from -100 °C to 100 °C, or from -100 °C to about 75 °C, or from -100 °C to 50 °C. In certain embodiments, the copolymer can have a T_{g} from -130 °C to 90 °C, or from -110 °C to 80 °C, or from -90 °C to 70 °C, or from -70 °C to 60 °C, or from -50 °C to t 50 °C.

The copolymer can also have a T_{g} in the lower or higher end of the range. For example, in some embodiments, the copolymer can have a T_{g} from -150 °C to 0 °C, or from -130 °C to -10 °C, or from -110 °C to -20 °C, or from -90 °C to t -30 °C. In other embodiments, the copolymer can have a T_{g} from 0 °C to 100 °C, or from 10 °C to 90 °C, or from 20 °C to 80 °C, or from 30 °C to 70 °C.

A higher T_{g} can increase the strength and rigidity of the copolymer. Strength and rigidity may be important for a stent formed of a polymeric material in certain applications, so that the stent can support the walls of a vessel.

On the other hand, a lower T_{g} can enhance the fracture toughness and flexibility of the copolymer, increase drug permeability, and improve drug-release control. Toughness and flexibility may be important for a range of aggressive applications of a stent, e.g., for a coated stent, such as overlapped stents, stent through stent delivery, and bifurcations. Polymers (e.g., certain glassy, semicrystalline polymers) with too high a T_{g} may be brittle under physiological conditions and fracture during application of the device, exhibiting little or no plastic deformation prior to failure.

The mechanical properties (e.g., strength, rigidity, toughness and flexibility) and physical properties (e.g., T_{g}, crystallinity/amorphousness, degradation rate, drug permeability and drug-release rate) of the copolymer can be tuned by appropriate selection of the polar and nonpolar monomer components; the number, ratio, and arrangement of the monomer components; the length or molecular weight, weight ratio, and arrangement of any segments or blocks of particular monomer component(s) within the copolymer; and any other substances physically or chemically attached to, blended with, or incorporated with the copolymer.

For example, a greater molar % of glycolide (GA), D-lactide (DLA), L-lactide (LLA), D,L-lactide (DLLA) or meso-lactide (MLA) in the copolymer can increase the strength and rigidity of the copolymer. On the other hand, a higher molar % of, e.g., propiolactone, valerolactone (VL), caprolactone (CL), trimethylene carbonate (TMC), dioxanone (DS), or acetals in the copolymer can increase the toughness and flexibility of the copolymer.

As another example, the mechanical and physical properties of the copolymer can be influenced by the selection of the particular relatively polar monomers. For example, copolymers derived from LLA tend be more crystalline than those derived from DLLA. Thus, if, e.g., a greater drug-release rate is desired, then a copolymer can be made more amorphous (i.e., less crystalline) by substituting DLLA for LLA or by incorporating a greater molar % content of DLLA in the copolymer.

Relatively polar and hydrophilic monomers such as GA, DLA, LLA, DLLA and MLA also enhance the degradation rate of the copolymer. Hydrophilic monomers increase the moisture content of the copolymer, which increases its degradation rate. Further, monomers that give acidic degradation products can increase the degradation rate of the copolymer, since the rate of the hydrolysis reaction tends to increase as the pH decreases.

As an example, for faster degradation the copolymer of the stent of the invention can contain GA units. When incorporated into a polymer, glycolide hydrolyzes faster than lactide, for the ester bond formed from glycolide is less sterically hindered than that formed from lactide. Further, glycolide units give acidic degradation products that can increase the degradation rate of a GA-containing copolymer.

Accordingly, in some embodiments of the copolymer of the stent, optionally in combination with one or more other embodiments described herein, the at least two polar monomers are selected from GA, DLA, LLA, DLLA and MLA, and the at least one nonpolar monomer is selected from VL, CL, TMC, DS, hydroxybutyrate (HB) and hydroxyvalerate (HV). In certain embodiments, the copolymer is a GA-containing terpolymer selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV). In a more specific embodiment, the copolymer is a GA- and CL-containing terpolymer selected from P(DLA-GA-CL), P(LLA-GA-CL), P(DLLA-GA-CL), and P(MLA-GA-CL).

The body's immune response to a polymeric material forming an implantable device may cause adverse side effects such as late stent thrombosis. To minimize or avoid such adverse immune responses, the copolymer of the invention is designed to completely or substantially completely degrade within 12 months. The copolymer can also be configured to degrade faster in cases where the implantable device is intended to accomplish its functions within a shorter period of time, e.g., locally delivering a drug up to 6 months or less. Accordingly, in certain embodiments, optionally in combination with one or more other embodiments described herein, the copolymer completely or substantially completely degrades within 9 months, or within 6 months, or within 3 months, or within 2 months, or within 1 month (i.e., 30 days).

The physical state of the copolymer can influence its degradation rate and drug permeability. Since a fluid (e.g., water) generally diffuses faster through an amorphous structure than through a crystalline structure, the copolymer can be configured to have a higher degree of amorphousness to increase its degradation rate and drug permeability. Increased water penetration into and water content in an amorphous polymer increases the degradation rate of the polymer and the diffusivity of a drug through the polymer.

Moreover, the physical state of the copolymer can influence its mechanical properties. A more crystalline copolymer can be stronger and more rigid. On the other hand, a more amorphous copolymer can be tougher and more flexible. A more amorphous copolymer can be more "elastomeric" or "rubbery" in that it can exhibit elastic deformation through a greater range of deformation. This characteristic may be desirable when the structure of an implantable device is anticipated to deform during the device's applications, e.g., when a stent that is crimped during delivery expands upon deployment.

To increase its toughness, flexibility, degradation rate and permeability to a hydrophobic drug, the copolymer can be configured to be more amorphous, i.e., be less crystalline. For example, the copolymer of the invention can be designed to have a degree of crystallinity of less than 50%. In some embodiments, the copolymer can have a lower degree of crystallinity depending on the properties desired for the copolymer. In certain embodiments, optionally in combination with one or more other embodiments described herein, the copolymer has a degree of crystallinity of less than 40%, or less than 30%, or less than 20%, or less than 10%, or less than 5%.

In some embodiments, optionally in combination with one or more other embodiments described herein, the biodegradable copolymer of the invention is amorphous. In certain embodiments, the amorphous copolymer has a degree of crystallinity of less than 20%, or less than 15%, or less than 10%, or less than 5%, or less than 3%.

The mechanical properties (e.g., strength, rigidity, toughness and flexibility) and physical properties (e.g., T_{g}, crystallinity/amorphousness, degradation rate, drug permeability, and drug-release rate) of the copolymer can be modified by appropriate selection of the relative amounts of the polar and nonpolar monomers. A higher molar % of the relatively polar and hydrophilic monomers GA, DLA, LLA, DLLA and MLA tends to increase the strength, rigidity, T_{g}, crystallinity and degradation rate of the copolymer (semicrystalline polymers derived from these kinds of monomers can degrade rapidly due to the chemical nature of these monomers). On the other hand, a greater molar % of the relatively nonpolar and hydrophobic monomers VL, CL, TMC, DS, HB and HV tends to lower the T_{g} and degree of crystallinity of the copolymer and increase its toughness, flexibility, degradation rate, miscibility with and permeability to a hydrophobic drug, and improve its controlled release of the hydrophobic drug. For example, a greater molar % of at least one such relatively nonpolar monomer can make the copolymer more amorphous.

In some embodiments, optionally in combination with one or more other embodiments described herein, the at least two relatively polar monomers and the at least one relatively nonpolar monomer each independently have a molar % content in the copolymer from 5% to 90%. In other embodiments, the molar % of each of the at least two relatively polar monomers and each of the at least one relatively nonpolar monomer independently is from 5% to about 85%, or from 7.5% to 80%, or from 10% to 75%, or from 12.5% to 70%, or from 15% to 65%. In certain embodiments, the molar % of each of the at least two relatively polar monomers independently is from 5% to 90% or from 5% to 85%, and the molar % of each of the at least one relatively nonpolar monomer independently is from 5% to 70%, or from 10% to 70%, or from 5% to 50%.

In further embodiments, the biodegradable copolymer is a terpolymer containing 5-50 molar % of GA and 5-70 molar % of a relatively nonpolar monomer such as CL. In narrower embodiments, the copolymer is a terpolymer containing 5-40 molar % of GA and 5-50 molar % of a relatively nonpolar monomer such as CL.

The number of units of particular polar and nonpolar monomers in the copolymer can be adjusted according to various factors such as the desired ratios of the monomers and the desired molecular weight of the copolymer or sections therein. According to the invention, each kind of polar and nonpolar monomers independently has from 10 to 5,000 units in the copolymer. In narrower embodiments, each kind of polar and nonpolar monomers independently has from 20 to 4,500 units, or from 30 to 4,000 units, or from 40 to 3,500 units, or from t 50 to 3,000 units in the copolymer.

For forming certain kinds of material (e.g., films, coatings, etc.), the entire copolymer may need to have sufficient molecular weight. Accordingly, in one embodiment, optionally in combination with one or more other embodiments described herein, the copolymer of the invention has a polymer number-average molecular weight (Mₙ) of at least 10 kDa. In other embodiments, the copolymer has an Mₙ of at least 20 kDa, or at least 30 kDa, or at least 40 kDa, or at least 50 kDa.

The range of Mₙ of the copolymer can also be influenced by the processing requirements for the particular kind of polymeric material. For example, a polymer with an Mₙ from 20 kDa to t 500 kDa may be more amenable to being processed into a coating. Thus, in some embodiments, optionally in combination with one or more other embodiments described herein, the inventive copolymer has an Mₙ from t 20 kDa to 500 kDa, or from 30 kDa to 500 kDa, or from 40 kDa to 500 kDa, or from 50 kDa to 500 kDa. According to the invention, the copolymer has an Mₙ from 10 kDa to 500 kDa.

The individual units of each different kind of polar and nonpolar monomers can be arranged in any manner (e.g., block, graft, random, alternating, etc.) in the copolymer, depending on the mechanical and physical properties desired for the copolymer. For example, the monomer units can be arranged in blocks or segments, where a block or a segment can contain one or more different kinds of monomers. The copolymer can contain a different number of blocks or segments - e.g., one, two, three, four or five blocks or segments. Certain blocks or segments can be the same as or different than other blocks or segments. Further, the monomer units or the blocks or segments can be arranged in an alternating or random manner. For example, a block or a segment can contain two or more different kinds of monomers arranged in an alternating or random manner. As another example, the blocks or segments themselves can be arranged in an alternating or random manner. The monomer units can also be arranged in a graft fashion or in any other manner as is known in the art.

If the monomer components are arranged in blocks or segments, the blocks or segments may or may not be miscible with each other. In one embodiment, the blocks or segments are partially or completely miscible with each other. In another embodiment, the blocks or segments are partially or completely immiscible with one another.

To form discrete phases which are indicative of an immiscible system, the blocks or segments need to be of a certain minimal size. Thus, in some embodiments, the blocks or segments of the copolymer each independently have an Mₙ of at least 2 kDa, or at least 3 kDa, or at least 5 kDa, or at least 10 kDa.

For a polymer with low permeability of a drug, a high drug/polymer ratio must be employed to get the drug to release. However, a high drug/polymer ratio can lead to a drug-release profile in which most of the drug is released as a burst, and the remaining portion of the drug is released very slowly. On the other hand, a low drug/polymer ratio may result in no or minimal drug release. A higher drug permeability of the polymer can allow better control of drug-release rates at reasonable drug-to-polymer ratios, e.g., where the amount of the polymer is greater than 50% by weight.

The copolymer of the invention is derived from at least one relatively nonpolar monomer (e.g., CL) that increases the miscibility of the copolymer with a hydrophobic drug such as rapamycin or a derivative thereof (e.g., everolimus). The increased miscibility enhances the copolymer's permeability to the hydrophobic drug and improves its controlled release of the drug. Moreover, the at least one relatively nonpolar monomer and its amount in the copolymer can be selected so as to lower the T_{g} of the copolymer and make the copolymer more amorphous, which would also enhance the diffusivity of a hydrophobic drug through the copolymer.

The enhanced permeability of the inventive copolymer to a hydrophobic drug allows for controlled release of the hydrophobic drug at lower drug-to-polymer (D:P) mass ratios. In some embodiments, optionally in combination with one or more other embodiments described herein, the mass ratio of a drug to the copolymer is from 1:1 to 1:10. In a narrower embodiment, the D:P mass ratio is from 1:1 to 1:5. In more specific embodiments, the D:P mass ratio is about 1:1, or 1:2, or 1:3, or 1:4, or 1:5. The D:P mass ratios described above apply independently to each of one or more hydrophobic drugs, hydrophilic drugs, biologically active agents, and any other kinds of drugs or agents that the inventive composition can comprise.

### Biocompatible Moieties

To enhance the biocompatibility of the copolymer, some embodiments of the inventive composition, optionally in combination with one or more other embodiments described herein, comprise the copolymer and one or more biologically compatible ("biocompatible") moieties. The biocompatible moieties can be physically or chemically attached to, blended with, or incorporated with the copolymer.

Examples of suitable biocompatible moieties include, but are not limited to, phosphoryl choline; poly(alkylene glycols), e.g., poly(ethylene glycol) (PEG), poly(ethylene oxide), poly(propylene glycol) (PPG), poly(tetramethylene glycol) and poly(ethylene oxide-co-propylene oxide); lactones and lactides, e.g., ε-caprolactone, β-butyrolactone, δ-valerolactone and glycolide; poly(N-vinyl pyrrolidone); poly(acrylamide methyl propane sulfonic acid) and salts thereof (AMPS and salts thereof); poly(styrene sulfonate); sulfonated dextran; polyphosphazenes; poly(orthoesters); poly(tyrosine carbonate); sialic acid; hyaluronic acid; hyaluronic acid having a stearoyl or palmitoyl substitutent group; copolymers of PEG with hyaluronic acid, hyaluronic acid-stearoyl or hyaluronic acid-palmitoyl; heparin; copolymers of PEG with heparin; a graft copolymer of poly(L-lysine) and PEG; and copolymers thereof. To ensure its renal clearance, the molecular weight of a polymeric biocompatible moiety may be 40 kDa or less, e.g., between 300 and 40,000 Daltons, or between 8,000 and 30,000 Daltons (e.g., 15,000 Daltons).

In certain embodiments, optionally in combination with one or more other embodiments described herein, the one or more biocompatible moieties are selected from phosphoryl choline, poly(ethylene oxide), poly(propylene glycol), poly(tetramethylene glycol), poly(ethylene oxide-co-propylene oxide), ε-caprolactone, β-butyrolactone, δ-valerolactone, glycolide, poly(N-vinyl pyrrolidone), poly(acrylamide methyl propane sulfonic acid) and salts thereof, poly(styrene sulfonate), sulfonated dextran, polyphosphazenes, poly(orthoesters), poly(tyrosine carbonate), sialic acid, hyaluronic acid and derivatives thereof, copolymers of poly(ethylene glycol) (PEG) with hyaluronic acid or derivatives thereof, heparin, copolymers of PEG with heparin, graft copolymers of poly(L-lysine) and PEG, and derivatives and copolymers thereof.

In some embodiments, optionally in combination with one or more other embodiments described herein, the one or more biocompatible moieties specifically cannot be one or more of any of the biocompatible moieties described herein.

### Non-Fouling Moieties

The body's reaction to foreign material could lead to adsorption of proteins on the surface of an implantable device, which could ultimately impair the device's functioning and result in adverse side effects such as thrombosis. A non-fouling moiety provides an implantable device with the ability to resist protein adsorption on its surface. Accordingly, some embodiments of the inventive composition, optionally in combination with one or more other embodiments described herein, comprise the biodegradable copolymer and one or more non-fouling moieties. The one or more non-fouling moieties can be physically or chemically attached to, blended with, or incorporated with the copolymer.

Examples of non-fouling moieties include, without limitation, poly(ethylene glycol) (PEG), poly(propylene glycol), polyethylene oxide, PLURONIC™ surfactants (polypropylene oxide-co-PEG), PEO-PPO surfactants (PLURONIC™ polyols, poly(ethylene oxide-co-propylene oxide)), poly(tetramethylene glycol), amino-terminated PEG, hydroxy functionalized poly(vinyl pyrrolidone), dextran, dextrin, sulfonated dextran, dermatan sulfate, silk-elastin block copolymers, sodium hyaluronate, hyaluronic acid, poly(2-hydroxyethyl methacrylate), dihydroxy poly(styrene sulfonate), poly(3-hydroxypropyl methacrylate), poly(3-hydroxypropyl methacrylamide), poly(alkoxy methacrylates), poly(alkoxy acrylates), polyarginine peptides (PAP) (e.g., R7), phosphoryl choline, heparin, chondroitan sulfate, glycosaminoglycans, chitosan, and derivatives thereof.

Silk and elastin both are natural proteins. Silk possesses great strength and elastin high flexibility. Their combination in a block copolymer makes the non-fouling moiety very strong and, at the same time, very flexible. Silk-elastin block copolymers can be obtained from Protein Polymer Technologies, Inc. of San Diego, California.

In certain embodiments, optionally in combination with one or more other embodiments described herein, the one or more non-fouling moieties are selected from polyethylene glycol (PEG), polypropylene glycol, Pluronic™ surfactants (polypropylene oxide-co-PEG), poly(2-hydroxyethyl methacrylate) (PHEMA), poly(vinyl alcohol) (PVA), polyalkene oxides, poly(n-propylmethacrylamide), poly(N-vinyl-2-pyrrolidone) (PVP), sulfonated polystyrene, dextran, sulfonated dextran, dextrin, hyaluronic acid, sodium hyaluronate, phosphoryl choline, and derivatives and copolymers thereof.

In some embodiments, optionally in combination with one or more other embodiments described herein, the one or more non-fouling moieties specifically cannot be one or more of any of the non-fouling moieties described herein.

The maximum molecular weight of the at least one non-fouling moiety or, if the non-fouling moiety itself is biodegradable, the maximum molecular weight of the largest fragment formed should be low enough so that it is small enough to pass through the kidneys of an animal (e.g., a human). Thus, in certain embodiments, the molecular weight of the at least one non-fouling moiety or its largest fragment is 40 kDa or less, or 30 kDa or less, or 20 kDa or less.

### Biobeneficial Materials

To improve the biological properties of an implantable device (e.g., enhance its biocompatibility and reduce protein adsorption on its surface), the device can be formed of a material comprising one or more biobeneficial materials. Therefore, some embodiments of the inventive composition, optionally in combination with one or more other embodiments described herein, comprise the biodegradable copolymer and one or more biobeneficial materials. The at least one biobeneficial material may be a polymeric material or a non-polymeric material, and may be biodegradable or non-degradable. In certain embodiments, the at least one biobeneficial material is flexible, biodegradable, biocompatible, non-toxic, non-antigenic and/or non-immunogenic. The at least one biobeneficial material can be physically or chemically attached to, blended with, or incorporated with the copolymer.

The at least one biobeneficial material, if polymeric, may have a relatively low T_{g}, e.g., a T_{g} less than or significantly less than that of the inventive copolymer. In an embodiment, the T_{g} of the biobeneficial material is below body temperature. Having a T_{g} below or significantly below that of the copolymer, the biobeneficial material would be relatively soft as compared to the copolymer. This attribute would, e.g., allow a layer of coating containing the biobeneficial material to fill any surface damages that may arise with an implantable device coated with a layer comprising the copolymer. For example, during radial expansion of a stent, a more rigid copolymer can crack or have surface fractures. A softer biobeneficial material can fill in the crack and fractures.

Examples of biobeneficial materials include, but are not limited to, polyethers (e.g., poly(ethylene glycol) (PEG)); poly(ether esters); co-poly(ether-esters) (e.g. PEO/PLA); polyalkylene oxides (e.g., poly(ethylene oxide) and poly(propylene oxide)); polyalkylene oxalates; polyphosphazenes; phosphoryl choline; choline; poly(aspirin); polymers and copolymers of hydroxyl bearing monomers such as hydroxyethyl methacrylate (HEMA), hydroxypropyl methacrylate (HPMA), hydroxypropylmethacrylamide, poly (ethylene glycol) acrylate (PEGA), PEG methacrylate, 2-methacryloyloxyethylphosphorylcholine (MPC) and n-vinyl pyrrolidone (VP); carboxylic acid bearing monomers such as methacrylic acid (MA), acrylic acid (AA), alkoxymethacrylate, alkoxyacrylate, and 3-trimethylsilylpropyl methacrylate (TMSPMA); copolymers of PEG such as poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), and poly(vinylidene fluoride)-PEG (PVDF-PEG); PLURONIC™ surfactants (polypropylene oxide-co-polyethylene glycol); poly(tetramethylene glycol); biomolecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharides, elastin, chitosan, and alginate; silicones; and combinations and copolymers thereof.

In certain embodiments, optionally in combination with one or more other embodiments described herein, the one or more biobeneficial materials are selected from fibrin; fibrinogen; cellulose and cellulose derivatives (e.g., cellulose acetate, cellulose butyrate, cellulose acetate butyrate, cellophane, cellulose nitrate, cellulose propionate, cellulose ethers, and carboxymethyl cellulose); starch; pectin; chitosan; elastin; gelatin; alginate and conjugates thereof (e.g., alginate-gelatin, alginate-collagen, alginate-laminin, alginate-elastin, alginate-collagen-laminin and alginate-hyaluronic acid); collagen and conjugates thereof; hyaluronan and derivatives thereof (e.g., methacrylate-modified hyaluronan and NHS ester-modified hyaluronan); hyaluronic acid; sodium hyaluronate; self-assembled peptides (SAPs) (e.g., AcN-RARADADARARADADA-CNH₂ (RAD 16-II), VKVKVKVKV-PP-TKVKVKVKV-NH₂ (MAX-1), and AcN-AEAEAKAKAEAEAKAK-CNH₂ (EAK 16-II)); and derivatives and copolymers thereof.

In another embodiment, the at least one biobeneficial material is a block copolymer having flexible poly(ethylene glycol) and poly(butylene terephthalate) blocks (PEG/PBT) (e.g., Poly Active™). PolyActive™ is intended to include AB, ABA, and BAB copolymers having such segments of PEG and PBT (e.g., poly(ethylene glycol)-block-poly(butylene terephthalate)-block-poly(ethylene glycol) (PEG-PBT-PEG)).

In some embodiments, optionally in combination with one or more other embodiments described herein, the one or more biobeneficial materials specifically cannot be one or more of any of the biobeneficial materials described herein.

### Biologically Active Agents

Other embodiments of the inventive composition, optionally in combination with one or more other embodiments described herein, comprise the biodegradable copolymer and at least one biologically active ("bioactive") agent. The at least one bioactive agent can be physically or chemically attached to, blended with, incorporated with or impregnated in the copolymer, and can include any substance capable of exerting a therapeutic, prophylactic or diagnostic effect for a patient. One of ordinary skill in the art would understand that the terms "bioactive agent" and "drug" can be used interchangeably in appropriate circumstances.

Examples of suitable bioactive agents include, but are not limited to, synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. The bioactive agents could be designed, e.g., to inhibit the activity of vascular smooth muscle cells. They could be directed at inhibiting abnormal or inappropriate migration and/or proliferation of smooth muscle cells to inhibit restenosis.

In certain embodiments, optionally in combination with one or more other embodiments described herein, the inventive composition comprises at least one biologically active agent selected from antiproliferative, antineoplastic, antimitotic, anti-inflammatory, antiplatelet, anticoagulant, antifibrin, antithrombin, antibiotic, antiallergic and antioxidant substances.

An antiproliferative agent can be a natural proteineous agent such as a cytotoxin or a synthetic molecule. Examples of antiproliferative substances include, but are not limited to, actinomycin D or derivatives and analogs thereof (manufactured by Sigma-Aldrich, or COSMEGEN available from Merck) (synonyms of actinomycin D include dactinomycin, actinomycin IV, actinomycin I₁, actinomycin X₁, and actinomycin C₁); all taxoids such as taxols, docetaxel, and paclitaxel and derivatives thereof; all olimus drugs such as macrolide antibiotics, rapamycin, everolimus, structural derivatives and functional analogues of rapamycin, structural derivatives and functional analogues of everolimus, FKBP-12 mediated mTOR inhibitors, biolimus, perfenidone, prodrugs thereof, co-drugs thereof, and combinations thereof. Examples of rapamycin derivatives include, but are not limited to, 40-*O*-(2-hydroxy)ethyl-rapamycin (generic name everolimus, available from Novartis), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus, manufactured by Abbott Labs.), prodrugs thereof, co-drugs thereof, and combinations thereof.

An anti-inflammatory drug can be a steroidal anti-inflammatory drug, a nonsteroidal anti-inflammatory drug (NSAID), or a combination thereof. Examples of anti-inflammatory drugs include, but are not limited to, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol, clobetasol propionate, clobetasone butyrate, clopirac, cloticasone propionate, cormethasone acetate, cortodoxone, deflazacort, desonide, desoximetasone, dexamethasone, dexamethasone acetate, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof

Alternatively, the anti-inflammatory agent can be a biological inhibitor of pro-inflammatory signaling molecules. Anti-inflammatory biological agents include antibodies to such biological inflammatory signaling molecules.

In addition, the bioactive agents can be other than antiproliferative or anti-inflammatory agents. The bioactive agents can be any agent that is a therapeutic, prophylactic or diagnostic agent. In some embodiments, such agents can be used in combination with antiproliferative or anti-inflammatory agents. These bioactive agents can also have antiproliferative and/or anti-inflammmatory properties or can have other properties such as antineoplastic, antimitotic, cystostatic, antiplatelet, anticoagulant, antifibrin, antithrombin, antibiotic, antiallergic, and/or antioxidant properties.

Examples of antineoplastics and/or antimitotics include, but are not limited to, paclitaxel (e.g., TAXOL® available from Bristol-Myers Squibb), docetaxel (e.g., Taxotere® from Aventis), methotrexate, azathioprine, vincristine, vinblastine, fluorouracil, doxorubicin hydrochloride (e.g., Adriamycin® from Pfizer), and mitomycin (e.g., Mutamycin® from Bristol-Myers Squibb).

Non-limiting examples of antiplatelet, anticoagulant, antifibrin, and antithrombin agents that can also have cytostatic or antiproliferative properties include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as ANGIOMAX (from Biogen), calcium channel blockers (e.g., nifedipine), colchicine, fibroblast growth factor (FGF) antagonists, fish oil (e.g., omega 3-fatty acid), histamine antagonists, lovastatin (a cholesterol-lowering drug that inhibits HMG-CoA reductase, brand name Mevacor® from Merck), monoclonal antibodies (e.g., those specific for platelet-derived growth factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetics, 4-amino-2,2,6,6-tetramethylpiperidine-1-oxyl (4-amino-TEMPO), estradiol, anticancer agents, dietary supplements such as various vitamins, and a combination thereof.

Examples of cytostatic substances include, but are not limited to, angiopeptin, angiotensin converting enzyme inhibitors such as captopril (e.g., Capoten® and Capozide® from Bristol-Myers Squibb), cilazapril and lisinopril (e.g., Prinivil® and Prinzide® from Merck).

Non-limiting examples of antiallergic agents include permirolast potassium. Examples of antioxidant substances include, but are not limited to, 4-amino-2,2,6,6-tetramethylpiperidine-1-oxyl (4-amino-TEMPO), tris(3,5-di-t-butyl-4-hydroxybenzyl)isocyanurate, 2,2'-methylenebis(4-methyl-6-t-butylphenol), 1,3,5-trimethyl-2,4,6-tris(3,5-di-t-butyl-4-hydroxybenzyl)benzene, butylhydroxytoluene, octadecyl 3,5-di-t-butyl-4-hydroxyhydrocinnamate, 4,4 methylenebis(2,6-di-butylphenol), p,p'-dioctyl diphenylamine, and 1,1,3-tris-(2-methyl-4-hydroxy-5-t-butylphenyl)butane.

Other bioactive agents include anti-infectives such as antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary vasodilators; peripheral and cerebral vasodilators; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and restenoic reducing agents.

Other biologically active agents that can be used include alpha-interferon, genetically engineered epithelial cells, tacrolimus and dexamethasone.

A "prohealing" drug or agent, in the context of a blood-contacting implantable device, refers to a drug or agent that has the property that it promotes or enhances re-endothelialization of arterial lumen to promote healing of the vascular tissue. The portion(s) of an implantable device (e.g., a stent) containing a prohealing drug or agent can attract, bind and eventually become encapsulated by endothelial cells (e.g., endothelial progenitor cells). The attraction, binding, and encapsulation of the cells will reduce or prevent the formation of emboli or thrombi due to the loss of the mechanical properties that could occur if the stent was insufficiently encapsulated. The enhanced re-endothelialization can promote the endothelialization at a rate faster than the loss of mechanical properties of the stent.

The prohealing drug or agent can be dispersed in the body of the bioabsorbable polymer substrate or scaffolding. The prohealing drug or agent can also be dispersed within a bioabsorbable polymer coating over a surface of an implantable device (e.g., a stent).

"Endothelial progenitor cells" refer to primitive cells made in the bone marrow that can enter the bloodstream and go to areas of blood vessel injury to help repair the damage. Endothelial progenitor cells circulate in adult human peripheral blood and are mobilized from bone marrow by cytokines, growth factors, and ischemic conditions. Vascular injury is repaired by both angiogenesis and vasculogenesis mechanisms. Circulating endothelial progenitor cells contribute to repair of injured blood vessels mainly via a vasculogenesis mechanism.

In some embodiments, the prohealing drug or agent can be an endothelial cell (EDC)-binding agent. In certain embodiments, the EDC-binding agent can be a protein, peptide or antibody, which can be, e.g., one of collagen type 1, a 23 peptide fragment known as single chain Fv fragment (scFv A5), a junction membrane protein vascular endothelial (VE)-cadherin, and combinations thereof. Collagen type 1, when bound to osteopontin, has been shown to promote adhesion of endothelial cells and modulate their viability by the down regulation of apoptotic pathways. S.M. Martin, et al., J. Biomed. Meter. Res., 70A:10-19 (2004). Endothelial cells can be selectively targeted (for the targeted delivery of immunoliposomes) using scFv A5. T. Volkel, et al., Biochimica et Biophysica Acta, 1663:158-166 (2004). Junction membrane protein vascular endothelial (VE)-cadherin has been shown to bind to endothelial cells and down regulate apoptosis of the endothelial cells. R. Spagnuolo, et al., Blood, 103:3005-3012 (2004).

In a particular embodiment, the EDC-binding agent can be the active fragment of osteopontin, (Asp-Val-Asp-Val-Pro-Asp-Gly-Asp-Ser-Leu-Ala-Try-Gly). Other EDC-binding agents include, but are not limited to, EPC (epithelial cell) antibodies, RGD peptide sequences, RGD mimetics, and combinations thereof.

In further embodiments, the prohealing drug or agent can be a substance or agent that attracts and binds endothelial progenitor cells. Representative substances or agents that attract and bind endothelial progenitor cells include antibodies such as CD-34, CD-133 and vegf type 2 receptor. An agent that attracts and binds endothelial progenitor cells can include a polymer having nitric oxide donor groups.

The foregoing biologically active agents are listed by way of example and are not meant to be limiting. Other biologically active agents that are currently available or that may be developed in the future are equally applicable.

In some embodiments, optionally in combination with one or more other embodiments described herein, the composition of the invention comprises at least one biologically active agent selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combination thereofs.

In other embodiments, optionally in combination with one or more other embodiments described herein, the inventive composition comprises at least one hydrophobic drug. In certain embodiments, the at least one hydrophobic drug is selected from rapamycin and derivatives thereof such as everolimus, biolimus, 40-*O*-(3-hydroxy)propyl-rapamycin, 40-*O*-[2-(2-hydroxy)ethoxy]ethyl-rapamycin, 40-*O*-tetrazole-rapamycin, and zotarolimus. In a particular embodiment, the hydrophobic drug is everolimus.

In some embodiments, optionally in combination with one or more other embodiments described herein, the at least one biologically active agent specifically cannot be one or more of any of the bioactive agents or drugs described herein.

### Bioactive Agent-Release Profile

If the stent according to the invention comprising the copolymer, contains one or more bioactive agents or drugs, the composition, material or structure can have any one or any combination of a pulse, burst and sustained release profile for each of the bioactive agent(s). For simplicity, the release profile of the bioactive agent(s) will be discussed in the context of a drug-delivery stent formed of a material comprising the copolymer.

In some embodiments, optionally in combination with one or more other embodiments described herein, the stent provides at least sustained release of each of the one or more bioactive agents. In certain embodiments, the stent provides sustained release of each of the bioactive agent(s) over a period up to 12 months, or up to 9 months, or up to 6 months, or up to 3 months, or up to 2 months, or up to 1 month.

As an example, the stent can be configured to have a pulse or burst release of a bioactive agent, followed by a sustained release of the same agent. The amount of the bioactive agent released during the pulse or burst phase can be adjusted depending on the needs of the particular therapeutic application. For example, it may be desirable to have a pulse or burst release of some amount of the bioactive agent to initially load up the treatment site with a sufficient amount of the agent, but not such a large amount of the agent that the agent enters systemic circulation and may cause adverse effects. After the initial pulse or burst release, the stent can be designed to provide sustained release of the bioactive agent over a period of time depending on the therapeutic needs (e.g., over a period of one month for an anti-proliferative drug or over a period of two months for an anti-inflammatory drug).

The term "pulse release" generally refers to a release profile of a bioactive agent that features a sudden surge in the release rate of the agent. The surge in the release rate of the agent would then disappear within a period of time. A more detailed definition of the term can be found in Encyclopedia of Controlled Drug Delivery, Edith Mathiowitz, Ed., Culinary and Hospitality Industry Publications Services, which is incorporated by reference in its entirety.

In some embodiments, the term "burst release" refers to *in vivo* release of a substantial or large amount of a bioactive agent from an implantable device (e.g., from a coating disposed over the device) in 15 days or less, e.g., within 7 to 14 days. In certain embodiments, a burst release delivers at least 30%, or at least 40%, or at least about 50%, or at least 60%, or at least 70%, or at least 80% of a bioactive agent in 15 days or less.

The term "sustained release" generally refers to a release profile of a bioactive agent that can include zero-order release, exponential decay, step-function release or other release profiles that carry over a period of time, e.g., ranging from several days to several weeks, several months or a couple of years. The terms "zero-order release", "exponential decay" and "step-function release" as well as other sustained release profiles are well known in the art. See, e.g., Encyclopedia of Controlled Drug Delivery, Edith Mathiowitz, Ed., Culinary and Hospitality Industry Publications Services.

The release rate of a bioactive agent can be tailored by various means. For example, the release rate of an agent can be tailored by the coating concentration of the agent and the equilibrium water uptake of the barrier if the barrier is formed of a hydrophobic, nonabsorable polymer or the absorption rate if the barrier is formed of an absorbable polymer.

In some embodiments where the implantable device is formed of a material comprising two or more bioactive agents, one of the agents or more than one of them can have any one or a combination of a pulse, burst or sustained release profile. The stent (e.g., the coating disposed thereover) can have a release profile that features a pulse or burst release of one or more agents together with a sustained release of the one or more agents. In an embodiment, the stent can be configured to have a profile of a pulse or burst release of a first agent and sustained release of the first agent and a second agent. In another embodiment, the stent can be designed to have a pulse or burst release of two agents followed by a sustained release of both agents. In yet another embodiment, the stent can be configured to provide a pulse release of one or more agents and optionally a sustained release of the same or different agents.

In further embodiments, optionally in combination with one or more other embodiments described herein, the stent (e.g., the coating disposed thereover) is capable of simultaneously releasing two or more bioactive agents. Simultaneous delivery means that there is at least some overlap in the release of the agents. Under this embodiment, one of the agents can be released first such as by pulse, burst, or sustained release so long as there is an overlap in release with the second agent.

A coating capable of simultaneously releasing two or more bioactive agents can have a variety of configurations. For example, the coating can have a layer that comprises a mixture of two agents, or it can have two layers, each of which comprises a particular bioactive agent and a polymer that may be the same as or different than the polymer in the other layer.

### Material and Coating

The composition used in the stent of the invention comprising the biodegradable copolymer is used to make a material of which an stent is formed. Such a material can comprise any combination of embodiments of the composition or copolymer described herein.

Accordingly, some embodiments used in the invention, optionally in combination with one or more other embodiments described herein, are drawn to a material containing any combination of embodiments of the composition comprising the biodegradable copolymer. For example, the composition forming the material can optionally contain at least one biocompatible moiety, at least one non-fouling moiety, at least one biobeneficial material, at least one biologically active agent, or a combination thereof.

The material used in the invention can be used to form a portion (e.g., of the body or the surface) of a stent or the whole device itself. For example, the material can be used to make a coating that is disposed over at least a portion of the stent.

Accordingly, some embodiments of the invention, optionally in combination with one or more other embodiments described herein, are directed to a stent comprising a coating containing any combination of embodiments of the composition comprising the biodegradable copolymer. For example, the composition forming the coating can optionally contain at least one biocompatible moiety, at least one non-fouling moiety, at least one biobeneficial material, at least one biologically active agent, or a combination thereof.

In some embodiments, optionally in combination with one or more other embodiments described herein, the biodegradable copolymer in the coating is derived from at least two polar monomers selected from GA, DLA, LLA, DLLA and MLA, and at least one nonpolar monomer selected from VL, CL, TMC, DS, HB and HV. In certain embodiments, the copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

In some embodiments, optionally in combination with one or more other embodiments described herein, the biodegradable copolymer in the coating has a degree of crystallinity of less than 50%, or less than 40%, or less than 30%, or less than 20%, or less than 10%, or less than 5%. In certain embodiments, the copolymer is amorphous. In other embodiments, the copolymer has a T_{g} from -150 °C to 100 °C, or from -100 °C to 100 °C, or from t -100 °C to 75 °C, or from -100 °C to 50 °C, or from -50 °C to 50 °C.

The coating can have a range of thickness and drug-to-polymer ratio depending on, e.g., the desired drug-release rate and degradation rate of the coating. In some embodiments, optionally in combination with one or more other embodiments described herein, the coating has a thickness of ≤ 20 micron, or ≤ 15 micron, or ≤ 10 micron, or ≤ 6 micron. In other embodiments, optionally in combination with one or more other embodiments described herein, the coating completely or substantially completely degrades within 12 months, or within 9 months, or within 6 months, or within 3 months, or within 2 months, or within 1 month.

If the coating comprises one or more bioactive agents or drugs, the coating can provide any one or any combination of a pulse, burst and sustained release of each of the bioactive agent(s) or drug(s). In some embodiments, optionally in combination with one or more other embodiments described herein, the coating provides sustained release of each of the bioactive agent(s) over a period up to 12 months, or up to 9 months, or up to 6 months, or up to 3 months, or up to 2 months, or up to 1 month.

In some embodiments, optionally in combination with one or more other embodiments described herein, the coating has a mass ratio of each of the drug(s) to the biodegradable copolymer independently ranging from 1:1 to 1:10, or from 1:1 to 1:5. In more specific embodiments, for each of the drug(s) the coating independently has a drug-to-copolymer mass ratio of 1:1, or 1:2, or 1:3, or 1:4, 1:5.

In further embodiments, optionally in combination with one or more other embodiments described herein, the coating comprises at least one bioactive agent or drug selected from selected from antiproliferative, antineoplastic, antimitotic, anti-inflammatory, antiplatelet, anticoagulant, antifibrin, antithrombin, antibiotic, antiallergic and antioxidant substances. In certain embodiments, the coating comprises at least one bioactive agent or drug selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combinations thereof.

### Structure of Coating

According to some embodiments of the invention, optionally in combination with one or more other embodiments described herein, a coating disposed over a stent can be a multi-layer structure that can include any of the following four layers or combination thereof:
(1) a primer layer;
(2) a drug-polymer layer (also referred to as a "reservoir" or "reservoir layer") or, alternatively, a polymer-free drug layer;
(3) a topcoat layer; and/or
(4) a finishing coat layer.

Each layer of a stent coating can be disposed over the stent by dissolving the polymer or a blend of polymers in a solvent, or a mixture of solvents, and disposing the resulting polymer solution over the stent by spraying or immersing the stent in the solution. After the solution has been disposed over the stent, the coating is dried by allowing the solvent to evaporate. The process of drying can be accelerated if the drying is conducted at an elevated temperature. The complete stent coating can be optionally annealed at a temperature between 40 °C and 150 °C for a period of time between 5 minutes and 60 minutes, if desired, to improve the thermodynamic stability of the coating.

To incorporate a bioactive agent (e.g., a drug) into the reservoir layer, the drug can be combined with the polymer solution that is disposed over the stent as described above. Alternatively, if it is desirable to have the stent coating with a fast drug-release rate, a polymer-free reservoir can be made. To fabricate a polymer-free reservoir, the drug can be dissolved in a suitable solvent or mixture of solvents, and the resulting drug solution can be disposed over the stent by spraying or immersing the stent in the drug-containing solution.

Instead of introducing a drug via a solution, the drug can be introduced as a colloid system, such as a suspension in an appropriate solvent phase. To make the suspension, the drug can be dispersed in the solvent phase using conventional techniques used in colloid chemistry. Depending on a variety of factors, e.g., the nature of the drug, those having ordinary skill in the art can select the solvent to form the solvent phase of the suspension, as well as the quantity of the drug to be dispersed in the solvent phase. Optionally, a surfactant can be added to stabilize the suspension. The suspension can be mixed with a polymer solution and the mixture can be disposed over the stent as described above. Alternatively, the drug suspension can be disposed over the stent without being mixed with the polymer solution.

The drug-polymer layer can be applied directly or indirectly over at least a portion of the stent surface to serve as a reservoir for at least one bioactive agent (e.g., drug) that is incorporated into the reservoir layer. The optional primer layer can be applied between the stent and the reservoir to improve the adhesion of the drug-polymer layer to the stent. The optional topcoat layer can be applied over at least a portion of the reservoir layer and serves as a rate-limiting membrane that helps to control the rate of release of the drug. In one embodiment, the topcoat layer can be essentially free from any bioactive agents or drugs. If the topcoat layer is used, the optional finishing coat layer can be applied over at least a portion of the topcoat layer for further control of the drug-release rate and for improving the biocompatibility of the coating. Without the topcoat layer, the finishing coat layer can be deposited directly on the reservoir layer.

The copolymer used in the invention derived from at least two polar monomers and at least one nonpolar monomer can be used to form any layer of the coating. In one embodiment, the copolymer forms the drug reservoir, or drug matrix, layer of the coating. In another embodiment, the copolymer forms the topcoat layer. In yet another embodiment, the copolymer forms the finishing coat layer. In still another embodiment, the copolymer forms the primer layer. In a further embodiment, the copolymer forms any combination of the primer, drug reservoir, topcoat and finishing coat layers of the coating.

The process of the release of a drug from a coating having both topcoat and finishing coat layers includes at least three steps. First, the drug is absorbed by the polymer of the topcoat layer at the drug-polymer layer/topcoat layer interface. Next, the drug diffuses through the topcoat layer using the void volume between the macromolecules of the topcoat layer polymer as pathways for migration. The drug then arrives at the topcoat layer/finishing layer interface. Finally, the drug diffuses through the finishing coat layer in a similar fashion, arrives at the outer surface of the finishing coat layer, and desorbs from the outer surface. At this point, the drug is released into the blood vessel or surrounding tissue. Consequently, a combination of the topcoat and finishing coat layers, if used, can serve as a rate-limiting barrier. The drug can be released by virtue of the degradation, dissolution, and/or erosion of the layer(s) forming the coating, or via migration of the drug through degradable and/or non-degradable polymeric layer(s) into a blood vessel or tissue.

In one embodiment, any or all of the layers of the stent coating can be made of biodegradable polymer(s), biostable polymer(s), or a combination thereof. In another embodiment, the outermost layer of the coating can be limited to biodegradable polymer(s), biostable polymer(s), or a combination thereof.

To illustrate in more detail, in a stent coating having all four layers described above (i.e., the primer, the reservoir layer, the topcoat layer and the finishing coat layer), the outermost layer is the finishing coat layer, which can be made of biodegradable polymer(s), biostable polymer(s), or a combination thereof. The remaining layers (i.e., the primer, the reservoir layer and the topcoat layer) optionally can also be fabricated of biodegradable polymer(s), biostable polymer(s), or a combination thereof. The polymer(s) in a particular layer may be the same as or different than those in any of the other layers.

If a finishing coat layer is not used, the topcoat layer can be the outermost layer and can be made of biodegradable polymer(s), biostable polymer(s), or a combination thereof. In this case, the remaining layers (i.e., the primer and the reservoir layer) optionally can also be fabricated of biodegradable polymer(s), biostable polymer(s), or a combination thereof. The polymer(s) in a particular layer may be the same as or different than those in any of the other layers.

If neither a finishing coat layer nor a topcoat layer is used, the stent coating could have only two layers - the primer and the reservoir. In such a case, the reservoir is the outermost layer of the stent coating and can be made of biodegradable polymer(s), biostable polymer(s), or a combination thereof. The primer optionally can also be fabricated of biodegradable polymer(s), biostable polymer(s), or a combination thereof. The two layers may be made from the same or different polymers.

Increased rate of degradation, erosion, absorption and/or resorption of biologically degradable, erodable, absorbable and /or resorbable polymer(s) can lead to an increased rate of release of a drug due to the gradual disappearance of the polymer(s) that form the reservoir, the topcoat layer, and/or the finishing coat layer. Through appropriate selection of biodegradable polymer(s), biostable polymer(s) or a combination thereof, a stent coating can be engineered to provide either fast or slow release of a drug, as desired. Those having ordinary skill in the art can determine whether a stent coating having slow or fast drug-release rate is advisable for a particular drug. For example, fast release may be recommended for stent coatings loaded with antimigratory drugs, which often need to be released within 1 to 2 weeks. For anti-proliferative and anti-inflammatory drugs, slower release may be desired, e.g., up to 30-day and 60-day release times, respectively.

Any layer of a stent coating can contain any amount of a bioabsorbable polymer and/or a biocompatible polymer, or a blend of more than one such polymer. Non-limiting examples of bioabsorbable polymers and biocompatible polymers include polyacrylates, e.g., poly(butyl methacrylate), poly(ethyl methacrylate), poly(ethyl methacrylate-co-butyl methacrylate), poly(acrylonitrile), poly(ethylene-co-methyl methacrylate), poly(acrylonitrile-co-styrene) and poly(cyanoacrylates); fluorinated polymers and/or copolymers, e.g., poly(vinylidene fluoride) and poly(vinylidene fluoride-co-hexafluoro propylene); poly(N-vinyl pyrrolidone); polydioxanone; polyorthoesters; polyanhydrides; poly(glycolic acid); poly(glycolic acid-co-trimethylene carbonate); polyphosphoesters; polyphosphoester urethanes; poly(amino acids); poly(trimethylene carbonate); poly(iminocarbonates); co-poly(ether-esters); polyalkylene oxalates; polyphosphazenes; biomolecules, e.g., fibrin, fibrinogen, cellulose, cellophane, starch, collagen, hyaluronic acid, and derivatives thereof (e.g., cellulose acetate, cellulose butyrate, cellulose acetate butyrate, cellulose nitrate, cellulose propionate, cellulose ethers, and carboxymethyl cellulose); polyurethanes; silicones; polyesters; polyolefins; polyisobutylene and ethylene-alphaolefin copolymers; vinyl halide polymers and copolymers, e.g., polyvinyl chloride; polyvinyl ethers, e.g., polyvinyl methyl ether; polyvinylidene chloride; polyvinyl ketones; polyvinyl aromatics, e.g., polystyrene; polyvinyl esters, e.g., polyvinyl acetate; copolymers of vinyl monomers with each other and olefins, e.g., poly(ethylene-co-vinyl alcohol) (EVAL); ABS resins; poly(ethylene-co-vinyl acetate); polyamides, e.g., Nylon 66 and polycaprolactam; alkyd resins; polycarbonates; polyoxymethylenes; polyimides; polyethers, epoxy resins; polyurethanes; rayon; rayon-triacetate; and copolymers thereof.

Any layer of a stent coating can also contain any amount of a non-degradable polymer, or a blend of more than one such polymer. Non-limiting examples of non-degradable polymers include methylmethacrylate, ethylmethacrylate, butylmethacrylate, 2-ethylhexylmethacrylate, laurylmethacrylate, hydroxyl ethyl methacrylate, polyethylene glycol (PEG) acrylate, PEG methacrylate, 2-methacryloyloxyethylphosphorylcholine (MPC) andN-vinyl pyrrolidone, methacrylic acid, acrylic acid, hydroxypropyl methacrylate, hydroxypropylmethacrylamide, 3-trimethylsilylpropyl methacrylate, and copolymers thereof.

### Implantable Device

The invention is directed to a stent formed of a material containing any combination of embodiments of the composition comprising the biodegradable copolymer as defined in claim 1. For example, the stent can be formed of a material comprising a composition that can optionally contain at least one biocompatible moiety, at least one non-fouling moiety, at least one biobeneficial material, at least one biologically active agent, or a combination thereof.

A portion of the stent or the whole stent itself can be formed of the material containing any combination of embodiments of the composition comprising the biodegradable copolymer as defined in claim 1. For example, a portion of the body or the whole body of the stent can be formed of the material. As another example, a coating containing any combination of embodiments of the composition comprising the biodegradable copolymer as defined in claim 1 can be disposed over at least a portion of the stent as defined in claim 1.

The coating is disposed over at least a portion of the stent. For example, the stent can be formed of a composition that can optionally contain at least one biocompatible moiety, at least one non-fouling moiety, at least one biobeneficial material, at least one biologically active agent, or a combination thereof.

The stent is formed of the polymeric material described in claim 1. The stent is formed of a material comprising a biodegradable copolymer that is derived from at least two polar monomers selected from GA, DLA, LLA, DLLA and MLA, and at least one nonpolar monomer selected from VL, CL, TMC, DS, HB and HV. As another example, the stent can be coated with a coating that has a thickness of ≤ about 20 micron, or ≤ about 15 micron, or ≤ about 10 micron, or ≤ about 6 micron.

As a further example, the stent can comprise a coating that completely or substantially completely degrades within 12 months, or within 9 months, or within 6 months, or within 3 months, or within 2 months, or within 1 month. As yet another example, the coating disposed over the stent can provide sustained release of one or more bioactive agents over a period up to 12 months, or up to 9 months, or up to 6 months, or up to 3 months, or up to 2 months, or up to 1 month.

The stent is a coronary stent or a peripheral stent.

The stent and its underlying structure can be of virtually any design. As an example, the stent can be of any size or shape that is suitable for the stent's intended functions. As another example, a portion of the stent, can be made of a metallic material, an alloy, a polymeric material, any other type of material, or a combination thereof, as is known in the art.

Non-limiting examples of metallic materials and alloys suitable for fabricating stents include cobalt-chromium alloys (e.g., ELGILOY), "L-605", stainless steel (316L), "MP35N," "MP20N," ELASTINITE (Nitinol), tantalum, tantalum-based alloys, nickel-titanium alloys, platinum, platinum-based alloys (e.g., platinum-iridium alloy), iridium, gold, magnesium, titanium, titanium-based alloys, zirconium-based alloys, or combinations thereof. "L-605" is a trade name for an alloy of cobalt, chromium, tungsten, nickel and iron available as Haynes 25 from Haynes International (Kokomo, Indiana). "L-605" consists of 51% cobalt, 20% chromium, 15% tungsten, 10% nickel and 3% iron. "MP35N" and "MP20N" are trade names for alloys of cobalt, nickel, chromium and molybdenum available from Standard Press Steel Co. (Jenkintown, Pennsylvania). "MP35N" consists of 35% cobalt, 35% nickel, 20% chromium and 10% molybdenum. "MP20N" consists of 50% cobalt, 20% nickel, 20% chromium and 10% molybdenum.

If a polymeric material as defined in claim 1 is used to make a portion (e.g., a coating) of the stent or the whole stent itself, the polymeric material can comprise any combination of embodiments of the composition used in the invention, besides the biodegradable copolymer of the invention as defined in claim 1, e.g. a blend of different types of polymers, a blend of polymer(s) and additional substance(s), or a combination thereof. Further, additional polymer(s) and/or additional substance(s) can be physically or chemically attached to the underlying copolymer forming the device or a portion thereof. The additional polymer(s) and/or additional substance(s) that can be physically or chemically attached to, blended with, or incorporated with the underlying copolymer include, but are not limited to, biocompatible polymers, bioabsorbable polymers, biocompatible moieties, non-fouling moieties, biobeneficial substances and materials, and bioactive agents. To enhance the mechanical characteristics (e.g., strength and rigidity) of an implantable device made substantially of a polymeric material, the device can be supported by additional structure(s) (e.g., struts in the case of stents made substantially of a polymeric material).

### Method of Fabricating Implantable Device

Other embodiments of the invention, optionally in combination with one or more other embodiments described herein, are drawn to a method of fabricating a stent as defined in claim 1. The method comprises forming the stent of a material containing the composition comprising the biodegradable copolymer as defined in claim 1. The composition can optionally contain at least one biocompatible moiety, at least one non-fouling moiety, at least one biobeneficial material, at least one biologically active agent, or a combination thereof.

Under the method, a portion of the stent or the whole stent itself is formed of the material containing the composition comprising the biodegradable copolymer as defined in claim 1. For example, the method can comprise depositing over at least a portion of the stent a coating containing any combination of embodiments of the composition comprising the biodegradable copolymer as defined in claim 1.

Accordingly, in some embodiments, the method comprises disposing over at least a portion of the stent a coating containing any combination of embodiments of the composition comprising the biodegradable copolymer as defined in claim 1. For example, the method comprises depositing over at least a portion of a stent a coating comprising a composition that can optionally contain at least one biocompatible moiety, at least one non-fouling moiety, at least one biobeneficial material, at least one biologically active agent, or a combination thereof.

The method can deposit a coating having a range of thickness over an implantable device. In certain embodiments, the method deposits over at least a portion of the implantable device a coating that has a thickness of ≤ 20 micron, or ≤ 15 micron, or ≤ 10 micron, or ≤ 6 micron.

The biodegradable copolymer used in the invention, and any other desired substances and materials, can be formed into a polymer construct, such as a tube or sheet that can be rolled or bonded to form a construct such as a tube. A stent is then be fabricated from the construct. For example, a stent can be fabricated from a tube by laser machining a pattern into the tube. In another embodiment, a polymer construct can be formed from the polymeric material used in the invention using an injection-molding apparatus.

Non-limiting examples of polymers that can be used to fabricate a stent include poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(L-lactide-co-glycolide), poly(D,L-lactide), poly(caprolactone), poly(trimethylene carbonate), polyethylene amide, polyethylene acrylate, poly(glycolic acid-co-trimethylene carbonate), co-poly(ether-esters) (e.g., PEO/PLA), polyphosphazenes, biomolecules (e.g., fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (e.g., polyvinyl chloride), polyvinyl ethers (e.g., polyvinyl methyl ether), polyvinylidene halides (e.g., polyvinylidene chloride), polyacrylonitrile, polyvinyl ketones, polyvinyl aromatics (e.g., polystyrene), polyvinyl esters (e.g., polyvinyl acetate), acrylonitrile-styrene copolymers, ABS resins, polyamides (e.g., Nylon 66 and polycaprolactam), polycarbonates, polyoxymethylenes, polyimides, polyethers, polyurethanes, rayon, rayon-triacetate, cellulose and derivates thereof (e.g., cellulose acetate, cellulose butyrate, cellulose acetate butyrate, cellophane, cellulose nitrate, cellulose propionate, cellulose ethers, and carboxymethyl cellulose), and copolymers thereof.

Additional representative examples of polymers that may be suited for fabricating a stent include ethylene vinyl alcohol copolymer (commonly known by the generic name EVOH or by the trade name EVAL), poly(butyl methacrylate), poly(vinylidene fluoride-co-hexafluoropropylene) (e.g., SOLEF 21508, available from Solvay Solexis PVDF of Thorofare, New Jersey), polyvinylidene fluoride (also known as KYNAR, available from ATOFINA Chemicals of Philadelphia, Pennsylvania), poly(tetrafluoroethylene-co-hexafluoropropylene-co-vinylidene fluoride), ethylene-vinyl acetate copolymers, and polyethylene glycol.

### Method of Treating or Preventing Disorders

A stent formed of a material containing the composition comprising the biodegradable copolymer as defined in claim 1 can be used to treat, prevent or diagnose various conditions or disorders. Examples of such conditions or disorders include, but are not limited to, vascular and related conditions or disorders such as atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, and arteriovenous anastamoses.

Accordingly, some embodiments of the invention, optionally in combination with one or more other embodiments described herein, are drawn to a method of treating, preventing or diagnosing a condition or disorder in a patient, comprising implanting in the patient a stent formed of a material containing the composition comprising the biodegradable copolymer as defined in claim 1. For example, the stent can be formed of a material comprising a composition as defined in claim 1 that can optionally contain at least one biocompatible moiety, at least one non-fouling moiety, at least one biobeneficial material, at least one biologically active agent, or a combination thereof.

In certain embodiments, optionally in combination with one or more other embodiments described herein, the method treats, prevents or diagnoses a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, and arteriovenous anastamoses. In a more specific embodiment, the condition or disorder is atherosclerosis, thrombosis, restenosis, vulnerable plaque, or a combination thereof.

A portion of the stent or the whole stent employed in the method is formed of a material containing the composition comprising the biodegradable copolymer, as described in claim 1. In some embodiments, a coating is disposed over at least a portion of the stent, wherein the coating contains the composition comprising the biodegradable copolymer as defined in claim 1. The coating can optionally contain at least one biocompatible moiety, at least one non-fouling moiety, at least one biobeneficial material, at least one biologically active agent, or a combination thereof.

The biodegradable copolymer in the coating is derived from at least two polar monomers selected from GA, DLA, LLA, DLLA and MLA, and at least one nonpolar monomer selected from VL, CL, TMC, DS, HB and HV. In certain embodiments, the copolymer is a GA-containing terpolymer selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV). In a more specific embodiment, the biodegradable copolymer in the coating is a GA- and CL-containing terpolymer selected from P(DLA-GA-CL), P(LLA-GA-CL), P(DLLA-GA-CL), and P(MLA-GA-CL).

The biodegradable copolymer derived from at least two polar monomers as defined in claim 1 and at least one nonpolar monomer as defined in claim 1 can be used to form any layer of the coating. In one embodiment, the copolymer forms the drug reservoir, or drug matrix, layer of the coating. In another embodiment, the copolymer forms the topcoat layer. In yet another embodiment, the copolymer forms the finishing coat layer. In still another embodiment, the copolymer forms the primer layer. In a further embodiment, the copolymer forms any combination of the primer, drug reservoir, topcoat and finishing coat layers of the coating.

In further embodiments of the method, the biodegradable copolymer in the coating disposed over the stent has a degree of crystallinity of less than 50%, or less than 40%, or less than 30%, or less than 20%, or less than 10%, or less than 5%. In certain embodiments, the copolymer is amorphous. In other embodiments, the copolymer has a T_{g} from -150 °C to 100 °C, or from -100 °C to 100 °C, or from -100 °C to 75 °C, or from -100 °C to 50 °C, or from -50 °C to 50 °C.

The coating over the stent that is used in the method can have a range of thickness and drug-to-polymer ratio depending on, e.g., the desired drug-release rate and degradation rate of the coating. In some embodiments, the coating has a thickness of ≤ 20 micron, or ≤ 15 micron, or ≤ 10 micron, or ≤ 6 micron. In other embodiments, the coating completely or substantially completely degrades within 12 months, or within 9 months, or within 6 months, or within 3 months, or within 2 months, or within 1 month.

The stent coated with the composition used in the invention can provide any one or any combination of a pulse, burst and sustained release of one or more bioactive agents or drugs. In some embodiments, the coated stent provides at least sustained release of each of the bioactive agent(s). In certain embodiments, the coated stent provides sustained release of each of the bioactive agent(s) over a period up to 12 months, or up to 9 months, or up to 6 months, or up to 3 months, or up to 2 months, or up to 1 month.

In some embodiments, the coating over the implantable stent has a mass ratio of each of the bioactive agent(s) to the biodegradable copolymer independently ranging from t 1:1 to 1:10, or from 1:1 to 1:5. In more specific embodiments, for each of the bioactive agent(s) the coating used in the invention independently has a bioactive agent-to-copolymer mass ratio or 1:1, or 1:2, or 1:3, or 1:4, or 1:5.

In some embodiments of the method, the coating over the stent comprises one or more bioactive agents selected from antiproliferative, antineoplastic, antimitotic, anti-inflammatory, antiplatelet, anticoagulant, antifibrin, antithrombin, antibiotic, antiallergic and antioxidant substances. In certain embodiments, the coating comprises at least one bioactive agent selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combinations thereof.

In other embodiments, the coating over the stent comprises one or more hydrophobic drugs. In certain embodiments, the coating comprises at least one hydrophobic drug selected from rapamycin and derivatives thereof such as everolimus, biolimus, 40-*O*-(3-hydroxy)propyl-rapamycin, 40-*O*-[2-(2-hydroxy)ethoxy]ethyl-rapamycin, 40-*O*-tetrazole-rapamycin, and zotarolimus. In a particular embodiment, the at least one hydrophobic drug is everolimus.

### Examples

The examples set forth below are shown for the sole purpose of further illustrating embodiments of the present invention and are in no way meant to limit the invention. The following prophetic examples are given to aid in understanding the invention, but it is to be understood that the invention is not limited to the particular materials or procedures of the examples.

### Synthesis of Biodegradable Copolymers

The biodegradable copolymer used in the invention can be prepared by any method of polymerization known in the art. Methods of polymerization include, but are not limited to, solution-based polymerization and melt-phase polymerization. In solution-based polymerization, all the reactive components involved in the polymerization reaction are partially or completely dissolved in a solvent.

The copolymer used in the invention can be synthesized by standard methods known to those having ordinary skill in the art, e.g., by ring-opening polymerization (ROP) with the corresponding monomers of the copolymer and using an initiator. ROP can be catalyzed by an organic or inorganic acid (e.g., a Lewis acid), an organic (e.g., a tertiary amine base) or inorganic base (e.g., a Lewis base), an organometallic reagent, and/or heat, if necessary and if compatible with the reactants and product(s) of the reaction. For example, zirconium catalysts such as zirconium acetylacetone, zinc catalysts such as diethylzinc and zinc L-lactate, and tin catalysts such as stannous octoate and tin triflates are particularly suitable for the synthesis of biodegradable polyesters.

In some embodiments, the initiator employed in the synthesis of the copolymer has at least one active end group that is a hydroxyl, amino or thiol group. If the initiator has only one active end group, then the polymer grows only at one end. On the other hand, if the initiator has two active end groups, then a polymerization reaction occurs at both ends of the polymer. In an embodiment, the initiator is a diol, in which one of the hydroxyl end groups may optionally be protected. In another embodiment, the initiator is a diamine, in which one of the amino end groups may optionally be protected. In yet another embodiment, the initiator is a dithiol, in which one of the thiol end groups may optionally be protected. In further embodiments, the dihydroxy, diamino or dithiol initiator is C₂-C₂₄ and contains an optionally substituted aliphatic, heteroaliphatic, cycloaliphatic, heterocycloaliphatic, aromatic or heteroaromatic group, or a combination thereof. In other embodiments, the initiator is a diol selected from 1,2-ethanediol, 1,3-propanediol, 1,4-butanediol, 1,5-pentanediol, 1,6-hexanediol, 1,7-heptanediol, 1,8-octanediol, diethylene glycol, triethylene glycol, tetraethylene glycol, poly(ethylene glycol), poly(propylene glycol), poly(tetramethylene glycol), and poly(caprolactone) diol.

Accordingly, it is disclosed a method of preparing the composition, comprising performing ring-opening polymerization (ROP) reactions with an initiator, at least two polar monomers and at least one nonpolar monomer, wherein:
the initiator has one or two active hydroxyl, amino or thiol end groups;
the initiator can do the initial ROP reaction with a polar monomer or a nonpolar monomer;
the ROP reaction with each different kind of monomer can occur in any order and for any number of times; and
a particular ROP reaction can occur in the presence of only one kind of monomer or in the presence of two or more different kinds of monomers.

Various embodiments of the composition comprising the biodegradable copolymer can be prepared by optionally:
blending or physically or chemically attaching at least one biocompatible moiety with or to the copolymer;
blending or physically or chemically attaching at least one non-fouling moiety with or to the copolymer;
blending or physically or chemically attaching at least one biobeneficial material with or to the copolymer; and/or
blending, physically or chemically attaching, or impregnating at least one biologically active agent with, to, or in the copolymer.

One example of the synthesis of a biodegradable copolymer is the synthesis of a P(CL-GA-DLLA) terpolymer via ROP in **Scheme 1.** In this example, mono-protected 1,6-hexanediol is the initiator. It initiates ROP with ε-caprolactone (CL) to form PCL. The hydroxyl end group of PCL then initiates ROP with glycolide (GA) to generate P(CL-GA). Similarly, the hydroxyl end group of P(CL-GA) in turn initiates ROP with D,L-lactide (DLLA) to furnish P(CL-GA-DLLA).

In the example illustrated in Scheme 1, it is understood that a particular ROP reaction with a particular kind of monomer can occur any number of times. Therefore, the variables m, n and p can be any integer ≥ 1. For example, each of these variables can independently be from 10 to 5,000, or from 20 to 4,500, or from 30 to 4,000, or from 40 to 3,500, or from 50 to 3,000. The order of ROP reactions depicted in Scheme 1 is merely illustrative. The ROP reactions involving the three different kinds of monomer can occur in any order. Moreover, a particular ROP reaction can be random by being performed in the presence of two or more different kinds of monomers.

If a particular ROP reaction with a particular kind of monomer occurs multiple times, a block or segment derived from that monomer can be created. The block or segment can be of a certain length or molecular weight, and can be arranged in a random or alternating fashion with another block or segment derived from another kind of monomer. Further, a block or segment can be derived from two or more different kinds of monomers, wherein each kind of monomer undergoes a particular ROP reaction a certain number of times. These ROP reactions can also alternate in forming a block or segment. In addition, a random block or segment can be created by conducting an ROP reaction in the presence of two or more different kinds of polymers any number of times, optionally conducting another ROP reaction in the presence of two or more other kinds of polymers any number of times, and so on.

If desired, the protected "left" end of the copolymer in Scheme 1 can remain protected after the polymerization reactions have been completed, and the hydroxyl group at the "right" end can be functionalized in any desired manner. Alternatively, deprotection of the left end of the copolymer allows this end to be functionalized in any desired fashion, with appropriate pre-protection of the right end group, if desired. The use of protecting (or blocking) groups in organic synthesis is well known in the art.

For example, both hydroxyl end groups can be conjugated to a dihydroxyaryl group to enhance the adhesion of the copolymer to a metal surface. The dihydroxyaryl group can contain, e.g., an *ortho*-dihydroxyphenyl moiety such as 1,2-dihydroxyphenyl and 3,4-dihydroxyphenyl. 3,4-Dihydroxyphenyl-containing compounds include, e.g., dopamine and 3,4-dihydroxyhydrocinnamic acid. Dopamine could be conjugated to the hydroxyl end groups of the copolymer, e.g., via coupling with 1,1'-carbonyldiimidazole. 3,4-Dihydroxyhydrocinnamic acid could be conjugated to the hydroxyl end groups, e.g., by conversion of the cinnamic acid to the N-succidimyl ester or by use of dicyclohexylcarbodiimide (DCC) and
4-(dimethylamino)pyridinium (DPTS). Alternatively, conjugation of the cinnamic acid could be effected via a Mitsunobu reaction using triphenylphosphine and diethyl azodicarboxylate (DEAD) or diisopropyl azodicarboxylate (DIAD). Conjugation of a dihydroxyaryl group to an active end group (e.g., a hydroxyl, amino or thiol group) could also be effected using other reagents and methods, as is known in the art.

As another example, either the left or the right hydroxyl end group, or both end groups, independently can be attached to a biocompatible moiety, a non-fouling moiety, a biobeneficial material, and/or a bioactive agent. Monomers different than those shown in Scheme 1 and bearing a protected side group can also be used in synthesizing the copolymer. After completion of the polymerization reactions, the side groups can be deprotected and functionalized as desired, e.g., by attaching them to a biocompatible moiety, a non-fouling moiety, a biobeneficial material, and/or a bioactive agent.

As a further example, either the left or the right hydroxyl end group, or both end groups, can undergo ROP with CL, GA, DLLA or a different polar or nonpolar monomer to further develop the left and/or right ends of the copolymer. For example, protection of the right end of the copolymer in Scheme 1 and deprotection of the left end permit the left end to be elaborated in further polymerization reactions. The polymerization reactions occurring at the left end can involve any kinds of monomers, can transpire any number of times, and can occur in any order and manner, as desired.

An initiator (e.g., the 1,6-hexanediol initiator in Scheme 1) can also initiate ROP as an unprotected diol, dithiol or diamine. In such a case, both the right and left ends of the polymer would be elaborated in the same way in the polymerization reactions.

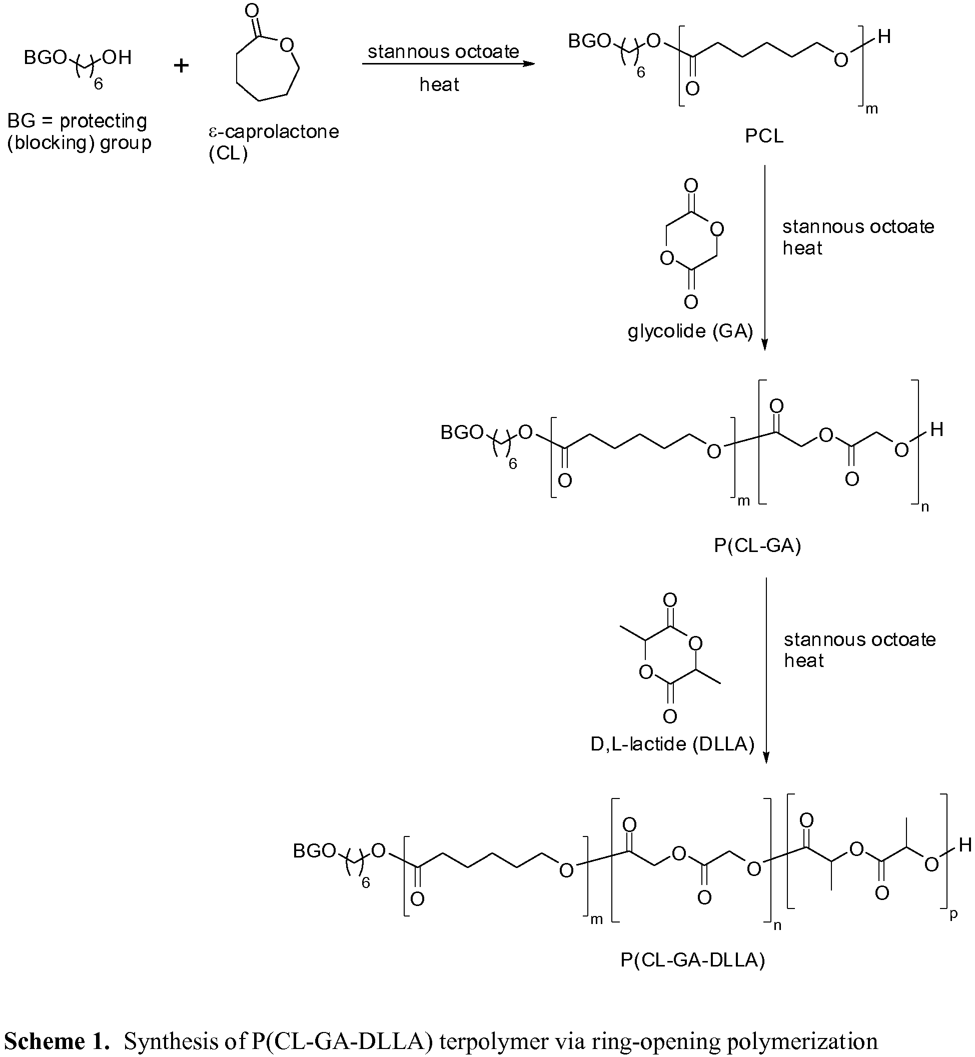

### Reference example 1. Controlled release of everolimus from P(DLLA-GA-CL) 60/15/25

A primer layer was formed on a stent from a primer solution containing about 2 weight % of P(DLLA-GA-CL) 60/15/25 (60 molar % DLLA, 15 molar % GA and 25 molar % CL) in 90/10 acetone/methyl isobutyl ketone (MIBK).

A solution containing about 2 weight % of P(DLLA-GA-CL) 60/15/25 and everolimus, at one of various drug-to-polymer (D:P) ratios, in 90/10 acetone/MIBK was prepared. The stent was mounted on a mandrel and spray-coated at a deposition rate of, e.g., about 10-20 microgram/pass. The stent was then dried in an oven at about 50 °C for about 30 minutes to evaporate the solvent. The dosage of everolimus was about 100 microgram/cm².

Drug release from the coated stent was analyzed by the "dipping" method. The stent was dipped in 10 mL of 0.1% sodium azide in porcine serum maintained at 37 ± 0.5 °C at a dipping rate of 40. The drug formulation was delivered into the release medium by a diffusion, dissolution and/or erosion process. Stent samples were withdrawn at 1- and 3-day intervals. At each time point, the stent was gently wiped with soft tissues and placed in 5 mL acetonitrile containing 0.02% BHT (butylated hydroxytoluene, specifically 2,6-bis-(t-butyl)-4-methylphenol). The stent was sonicated for 30 minutes to extract the drug. Samples (2 mL) were centrifuged at 13,000 rpm for 5 minutes for separation of any particulates, and analyzed by the optimized HPLC method. The amount of drug remaining on the stent after dipping was determined and the percent release of drug into the release medium was calculated using the assay/drug content values.

**Table 1** shows the release of everolimus from stents coated with P(DLLA-GA-CL) 60/15/25 at various conditions. The dosage of everolimus in all these examples was about 100 microgram/cm².

**Table 1. Release of everolimus from P(DLLA-GA-CL) 60/15/25**

| D:P Ratio | % Drug Release, Day 1 (n = 3) | % Drug Release, Day 3 (n = 3) |
|---|---|---|
| 1:3 | 66.7 ± 3.2 | 94.5 ± 0.7 |
| 1:5 | 56.5 ± 2.2 | 86.6 ± 0.5 |
| 1:3 with topcoat | 41.6 ± 2.6 | 77.4 ± 2.9 |

The results in Table 1 demonstrate that stents coated with P(DLLA-GA-CL) 60/15/25 terpolymers provide controlled release of everolimus and that the drug's release rate can be controlled by adjusting various factors such as, e.g., the D:P ratio and whether or not a topcoat layer is also applied.

### Reference example 2. Controlled release of everolimus from P(LLA-GA-CL) terpolymers

Similar procedures as those above were used to study the release of everolimus from drug-laden stents coated with P(LLA-GA-CL) terpolymers containing various molar percentages of LLA, GA and CL. **Figure 1** depicts the release of everolimus from stents coated with P(LLA-GA-CL) terpolymers, where the D:P ratio was 1:3 and the dosage of everolimus was about 100 microgram/cm². **Table 2** lists some of the results shown in Figure 1.

**Table 2. Release of everolimus from P(LLA-GA-CL) terpolymers, D:P = 1.3**

| Molar % of LLA/GA/CL | % Drug Release, Day 1 (n = 3) | % Drug Release, Day 3 (n = 3) |
|---|---|---|
| 40/30/30 | 88 ± 4.2 | 97.4 ± 1.5 |
| 50/25/25 | 58.6 ± 3 | 82.3 ± 2.3 |
| 60/15/25 | 47.4 ± 1.3 | 67.4 ± 4.5 |

As can be seen from Figure 1 and Table 2, stents coated with P(LLA-GA-CL) terpolymers provide controlled release of everolimus, and the drug's release rate can be controlled by adjusting various factor such as the molar % content of the LLA, GA and CL monomer components. Further, the results with P(DLLA-GA-CL) 60/15/25 and P(LLA-GA-CL) 60/15/25, both at a D:P ratio of 1:3, show that the release rate of a hydrophobic drug such as everolimus can also be controlled by the selection of the relatively polar monomer components - in this case, DLLA as compared to LLA.

While particular embodiments of the present invention have been shown and described, it will be obvious to those skilled in the art that changes and modifications can be made thereto without departing from the invention in its broader aspects. Therefore, the appended claims are to encompass within their scope all such changes and modifications as fall within the true spirit and scope of the present invention.

It is understood that if a substance can have more than one stereochemistry and/or regiochemistry at one or more stereocenters and/or regiocenters and the stereochemistry and/or regiochemistry of the substance at the one or more stereocenters and/or regiocenters are not indicated, the scope of the present invention encompasses all possible stereoisomers (e.g., enantiomers, diastereomers, etc.) and/or regioisomers of that substance.

## Claims

1. A stent formed of a composition comprising a biodegradable copolymer,
the biodegradable copolymer being derived from three or more monomers;
wherein the biodegradable copolymer is derived from:
at least two monomers selected from glycolide (GA), D-lactide (DLA), L-lactide (LLA), D,L-lactide (DLLA), and meso-lactide (MLA); and
at least one monomer selected from valerolactone (VL), caprolactone (CL), trimethylene carbonate (TMC), dioxanone (DS), hydroxybutyrate (HB), and hydroxyvalerate (HV);
wherein the stent is a coronary stent or a peripheral stent;
wherein the biodegradable copolymer has a Tg from -150 °C to 100 C;
wherein the biodegradable copolymer has a polymer number-average molecular weight (Mn) from 10 kDa to 500 kDa;
wherein the biodegradable copolymer completely degrades, which is the loss of at least 95 % of its mass, substantially completely degrades, which is the loss of at least 75% of its mass, within 12 months, when exposed to physiological conditions;
and
wherein for the biodegradable copolymer each kind of monomer independently has from 10 to 5,000 units in the copolymer; and the molar % of each kind of monomer independently is from 5% to 90%.

2. The stent of claim 1, wherein the biodegradable copolymer
has a Tg from -100 °C to 100 °C; and
has an Mn from 20 kDa to 500 kDa;
and wherein:
each kind of monomer independently has from 50 to 3,000 units in the copolymer.

3. The stent of claim 1, wherein the glass transition temperature of the biodegradable copolymer is from - 150 °C to 0 °C.

4. The stent of claim 1, wherein the biodegradable copolymer has a degree of crystallinity of less than 30%.

5. The stent of claim 1, wherein the biodegradable polymer is derived from at least two monomers selected from D-lactide (DLA), L-lactide (LLA), D,L-lactide (DLLA), and meso-lactide (MLA); and at least one monomer selected from valerolactone (VL), caprolactone (CL), trimethylene carbonate (TMC), dioxanone (DS), hydroxybutyrate (HB), and hydroxyvalerate (HV).

6. The stent of claim 1, wherein the biodegradable copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GADS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

7. The stent of claim 1, wherein the composition:
further comprises at least one biologically active agent selected from antiproliferative, antineoplastic, antimitotic, anti-inflammatory, antiplatelet, anticoagulant, antifibrin, antithrombin, antibiotic, antiallergic and antioxidant substances; and
has one or a combination of a pulse, burst and sustained release profile for each of the at least one biologically active agents.

8. The stent of claim 7, wherein the at least one biologically active agent is selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-O-(2-hydroxy)ethyl-rapamycin (everolimus), 40-O-(2-ethoxy)ethyl-rapamycin (biolimus), 40-O-(3-hydroxy)propyl-rapamycin, 40-O-[2-(2-hydroxy)ethoxy]ethyl-rapamycin, 40-O-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combinations thereof.

9. The stent of claim 7, wherein the at least one biologically active agent has a sustained release over a period up to 12 months.

10. The stent of claim 7, wherein the at least one biologically active agent has a sustained release over a period up to 9 months.

11. The stent of claim 7, wherein the at least one biologically active agent has a sustained release over a period up to 6 months.

12. The stent of claim 1, where the composition further comprises one or more components selected from biocompatible moieties, non-fouling moieties, biobeneficial materials, and combinations thereof.

13. The stent of claim 12, wherein:
the one or more biocompatible moieties are selected from phosphoryl choline, poly(ethylene oxide), poly(propylene glycol), poly(tetramethylene glycol), poly(ethylene oxide-co-propylene oxide), caprolactone, B-butyrolactone, valérolactone, glycolide, poly(N-vinyl pyrrolidone), poly(acrylamide methyl propane sulfonic acid) and salts thereof, poly(styrene sulfonate), sulfonated dextran, polyphosphazenes, poly(orthoesters), poly(tyrosine carbonate), sialic acid, hyaluronic acid and derivatives thereof, copolymers of poly(ethylene glycol) (PEG) with hyaluronic acid or derivatives thereof, heparin, copolymers of PEG with heparin, graft copolymers of poly(L-lysine) and PEG, and derivatives and copolymers thereof;
the one or more non-fouling moieties are selected from polyethylene glycol, polypropylene glycol, Pluronic™ surfactants, poly(2-hydroxyethyl methacrylate), poly(vinyl alcohol), polyalkene oxides, poly(n-propylmethacrylamide), poly(N-vinyl-2-pyrrolidone), sulfonated polystyrene, dextran, sulfonated dextran, dextrin, hyaluronic acid, sodium hyaluronate, phosphoryl choline, and derivatives and copolymers thereof; and
the one or more biobeneficial materials are selected from fibrin, fibrinogen, cellulose and derivatives thereof, starch, pectin, chitosan, elastin, gelatin, alginate and conjugates thereof, collagen and conjugates thereof, hyaluronan and derivatives thereof, hyaluronic acid, sodium hyaluronate, self-assembled peptides, and derivatives and copolymers thereof.

14. The stent of claim 1, further comprising a coating comprising biodegradable polymer(s), biostable polymer(s), or a combination thereof.

15. A stent as defined in claim 1, for use in the treatment or prevention of a condition or disorder in a patient selected from the group consisting of atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, and arteriovenous anastamoses.

16. The stent of claim 8, wherein the at least one biologically active agent is selected from paclitaxel, docetaxel, estradiol, dexamethasone, clobetasol, 4-amino-2,2,6,6-tetramethylpiperidine-1-oxyl (4-amino-TEMPO), tacrolimus (FK-506), rapamycin (sirolimus), 40-O-(2-hydroxy)ethyl-rapamycin (everolimus), 40-O-(2-ethoxy)ethyl-rapamycin (biolimus), 40-O-(3-hydroxy)propyl-rapamycin, 40-O-[2-(2-hydroxy)-ethoxy]-ethyl-rapamycin, 40-0-tetrazole-rapamycin, 40-epi-(N1-tetrazolyl)-rapamycin (zotarolimus), pimecrolimus, imatinib mesylate, midostaurin, and combinations thereof.

## Patentansprüche

1. Ein Stent bestehend aus einer Zusammensetzung umfassend ein biologisch abbaubares Copolymer,
wobei das biologisch abbaubare Copolymer von drei oder mehr Monomeren abgeleitet ist;
wobei das biologisch abbaubare Copolymer abgeleitet ist von:
mindestens zwei Monomeren, die ausgewählt aus Glycolid (GA), D-Lactid (DLA), L-Lactid (LLA), D,L-Lactid (DLLA), und meso-Lactid (MLA) sind; und
mindestens einem Momomer, das ausgewählt aus Valerolacton (VL), Caprolacton (CL), Trimethylencarbonat (TMC), Dioxanon (DS), Hydroxybutyrat (HB), und Hydroxyvalerat (HV) ist;
wobei der Stent ein koronarer Stent oder ein peripherer Stent ist;
wobei das biologisch abbaubare Copolymer eine Tg von -150 °C bis 100 °C hat;
wobei das biologisch abbaubare Copolymer ein Zahlenmittel des Molekulargewichts (Mn) des Polymers von 10 kDa bis 500 kDa hat;
wobei das biologisch abbaubare Copolymer sich vollständig abbaut, was einem Verlust von mindestens 95 % seiner Masse entspricht, sich im Wesentlichen vollständig abbaut, was dem Verlust von mindestens 75% seiner Masse entspricht, innerhalb von 12 Monaten, wenn es physiologischen Bedingungen ausgesetzt wird;
und
wobei für das biologisch abbaubare Copolymer jeder Monomerentyp unabhängig von 10 bis 5.000 Einheiten im Copolymer hat; und das molare % von jedem Monomerentyp unabhängig von 5% bis 90% reicht.

2. Der Stent des Anspruchs 1, wobei das biologisch abbaubare Copolymer eine Tg von -100 °C bis 100 °C hat; und
ein Mn von 20 kDa bis 500 kDa hat;
und wobei:
jeder Monomerentyp unabhängig von 50 bis 3.000 Einheiten im Copolymer hat.

3. Der Stent des Anspruchs 1, wobei die Glasübergangstemperatur des biologisch abbaubaren Copolymers von - 150 °C bis 0 °C reicht.

4. Der Stent des Anspruchs 1, wobei das biologisch abbaubare Copolymer einen Kristallinitätsgrad von weniger als 30% hat.

5. Der Stent des Anspruchs 1, wobei das biologisch abbaubare Polymer von mindestens zwei Monomeren ausgewählt aus D-Lactid (DLA), L-Lactid (LLA), D,L-Lactid (DLLA), und meso-Lactid (MLA) und mindestens einem Monomer ausgewählt aus Valerolacton (VL), Caprolacton (CL), Trimethylencarbonat (TMC), Dioxanon (DS), Hydroxybutyrat (HB), und Hydroxyvalerat (HV) abgeleitet ist.

6. Der Stent des Anspruchs 1, wobei das biologisch abbaubare Copolymer ausgewählt ist aus P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), und P(MLA-GA-HV).

7. Der Stent des Anspruchs 1, wobei die Zusammensetzung:
weiterhin mindestens einen biologischen Wirkstoff ausgewählt aus antiproliferativen, antineoplastischen, antimitotischen, entzündungshemmenden, antithrombozytären, gerinnungshemmenden, antifibrin, antithrombinen, antibiotischen, antiallergischen und antioxidativen Substanzen umfasst; und
eine oder eine Kombination von einem pulsierten, explosionsartigen ("burst") und anhaltenden Freisetzungsprofil für jeden des mindestens einen biologischen Wirkstoffs hat.

8. Der Stent des Anspruchs 7, wobei der mindestens eine biologische Wirkstoff ausgewählt ist aus Paclitaxel, Docetaxel, Estradiol, Dexamethason, Clobetasol, NO-Donatoren, Superoxid-Dismutasen, Superoxiddismutase-Mimetika, 4-Amino-2,2,6,6-tetramethylpiperidin-1-oxyl (4-amino-TEMPO), Tacrolimus (FK-506), Rapamycin (Sirolimus), Rapamycin -Derivaten, 40-O-(2-Hydroxy)ethyl-rapamycin (Everolimus), 40-O-(2-Ethoxy)ethyl-rapamycin (Biolimus), 40-O-(3-Hydroxy)propyl-rapamycin, 40-O-[2-(2-Hydroxy)ethoxy]ethyl-rapamycin, 40-O-Tetrazolrapamycin, 40-epi-(N1-Tetrazolyl)-rapamycin (Zotarolimus), Pimecrolimus, Imatinibmesylat, Midostaurin, Antikörpern, die Vorläuferzellen auffangen, heilungsfördemden Arzneimitteln, Prodrugs davon, Codrugs davon, und Kombinationen davon.

9. Der Stent des Anspruchs 7, wobei der mindestens eine biologische Wirkstoff eine anhaltende Freisetzung über einen Zeitraum von bis 12 Monaten hat.

10. Der Stent des Anspruchs 7, wobei der mindestens eine biologische Wirkstoff eine anhaltende Freisetzung über einen Zeitraum von bis 9 Monaten hat.

11. Der Stent des Anspruchs 7, wobei der mindestens eine biologische Wirkstoff eine anhaltende Freisetzung über einen Zeitraum von bis 6 Monaten hat.

12. Der Stent des Anspruchs 1, wobei die Zusammensetzung weiterhin eine oder mehrere Komponenten umfasst, die ausgewählt aus biokompatiblen Resten, nicht ablagernden Resten, biologisch vorteilhaften Stoffen, und Kombinationen davon sind.

13. Der Stent des Anspruchs 12, wobei:
der eine oder die mehreren biokompatiblen Resten ausgewählt sind aus Phosphorylcholin, Polyethylenoxid, Polypropylenglycol, Polytetramethylenglycol, Poly(Ethylenoxid-co-Propylenoxid), Caprolacton, β-Butyrolacton, Valerolacton, Glycolid, Poly-N-vinylpyrrolidon, Poly(Acrylamid-methyl-Propansulfonsäure) und Salzen davon, Polystyrensulfonat, sulfoniertem Dextran, Polyphosphazenen, Polyorthoestern, Polytyrosincarbonat, Sialsäure, Hyaluronsäure und Derivaten davon, Copolymeren von Polyethylenglycol (PEG) mit Hyaluronsäure oder Derivaten davon, Heparin, Copolymeren von PEG mit Heparin, Pfropfcopolymern von Poly-L-Lysin und PEG, und Derivaten und Copolymeren davon;
wobei der eine oder die mehreren nicht ablagernden Resten ausgewählt sind aus Polyethylenglycol, Polypropylenglycol, Pluronic™ Tensiden, Poly(2-hydroxyethyl methacrylat), Polyvinylalkohol, Polyalkenoxiden, Poly(n-propylmethacrylamid), Poly(N-vinyl-2-pyrrolidon), sulfoniertem Polystyren, Dextran, sulfoniertem Dextran, Dextrin, Hyaluronsäure, Natriumhyaluronat, Phosphorylcholin, und Derivaten und Copolymeren davon; und
wobei der eine oder die mehreren biologisch vorteilhaften Stoffe ausgewählt sind aus Fibrin, Fibrinogen, Cellulose und Derivaten davon, Stärke, Pectin, Chitosan, Elastin, Gelatine, Alginat und Konjugaten davon, Kollagen und Konjugaten davon, Hyaluronan und Derivaten davon, Hyaluronsäure, Natriumhyaluronat, selbstorganisierenden Peptiden, und Derivaten und Copolymeren davon.

14. Der Stent des Anspruchs 1, weiterhin umfassend eine Beschichtung umfassend biologisch abbaubares Polymer bzw. biologisch abbaubare Polymere, biostabiles Polymer bzw. biostabile Polymere, oder eine Kombination davon.

15. Ein Stent wie in Anspruch 1 definiert, zur Verwendung bei der Behandlung oder Prävention von einem Zustand oder einer Störung bei einem Patienten ausgewählt aus der Gruppe bestehend aus Atherosclerose, Thrombose, Restenose, Hämorrhagien, Gefäßdisektion, Gefäßperforation, Gefäßaneurysma, vulnerabler Plaque, chronischem Totalverschluss, offenem Foramen Ovale, Claudicatio, anastomotischer Proliferation für Venen- und künstliche Transplantate, und arteriovenöser Anastomose.

16. Der Stent des Anspruchs 8, wobei der mindestens eine biologische Wirkstoff ausgewählt ist aus Paclitaxel, Docetaxel, Estradiol, Dexamethason, Clobetasol, 4-Amino-2,2,6,6-tetramethylpiperidin-1-oxyl (4-amino-TEMPO), Tacrolimus (FK-506), Rapamycin (Sirolimus), 40-O-(2-Hydroxy)ethyl-rapamycin (Everolimus), 40-O-(2-Ethoxy)ethyl-rapamycin (Biolimus), 40-O-(3-Hydroxy)propyl-rapamycin, 40-O-[2-(2-Hydroxy)ethoxy]ethyl-rapamycin, 40-O-Tetrazol-rapamycin, 40-epi-(N1-Tetrazolyl)-rapamycin (Zotarolimus), Pimecrolimus, Imatinibmesylat, Midostaurin und Kombinationen davon.

## Revendications

1. Un stent formé d'une composition comprenant un copolymère biodégradable,
le copolymère biodégradable étant dérivé de trois ou plusieurs monomères ;
dans lequel le copolymère biodégradable est dérivé de :
au moins deux monomères choisis parmi le glycolide (GA), le D-lactide (DLA), le L-lactide (LLA), le D,L-lactide (DLLA), et le méso-lactide (MLA) ; et
au moins un monomère choisi parmi la valérolactone (VL), la caprolactone (CL), le carbonate de triméthylène (TMC), la dioxanone (DS), l'hydroxybutyrate (HB), et l'hydroxyvalérate (HV) ;
dans lequel le stent est un stent coronaire ou un stent périphérique ;
dans lequel le copolymère biodégradable a une Tg de -150 °C à 100 °C ;
dans lequel le copolymère biodégradable a une masse moléculaire de polymère moyenne en nombre (Mn) de 10 kDa à 500 kDa ;
dans lequel le copolymère biodégradable se dégrade complètement, ce qui équivaut à la perte d'au moins 95 % de sa masse, se dégrade de façon essentiellement complète, ce qui équivaut à la perte d'au moins 75% de sa masse, dans 12 mois, lorsqu'il est exposé à des conditions physiologiques ;
et
dans lequel pour le copolymère biodégradable chaque type de monomère a indépendamment de 10 à 5.000 unités dans le copolymère ; et le % molaire de chaque type de monomère est indépendamment de 5% à 90%.

2. Le stent de la revendication 1, dans lequel le copolymère biodégradable
a une Tg de -100 °C à 100 °C ; et
a une Mn de 20 kDa à 500 kDa ;
et dans lequel :
chaque type de monomère a indépendamment de 50 à 3.000 unités dans le copolymère.

3. Le stent de la revendication 1, dans lequel la température de transition vitreuse du copolymère biodégradable est de - 150 °C à 0 °C.

4. Le stent de la revendication 1, dans lequel le copolymère biodégradable a un taux de cristallinité de moins de 30%.

5. Le stent de la revendication 1, dans lequel le polymère biodégradable est dérivé d'au moins deux monomères choisis parmi le D-lactide (DLA), le L-lactide (LLA), le D,L-lactide (DLLA), et le méso-lactide (MLA) ; et au moins un monomère choisi parmi la valérolactone (VL), la caprolactone (CL), le carbonate de triméthylène (TMC), la dioxanone (DS), l'hydroxybutyrate (HB), et l'hydroxyvalérate (HV).

6. Le stent de la revendication 1, dans lequel le copolymère biodégradable est choisi parmi P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), et P(MLA-GA-HV).

7. Le stent de la revendication 1, dans lequel la composition :
comprend en outre au moins un agent biologiquement actif choisi parmi des substances antiprolifératives, antinéoplastiques, antimitotiques, anti-inflammatoires, antiplaquettaires, anticoagulantes, antifibrines, antithrombines, antibiotiques, antiallergiques et antioxydantes ; et
a un ou une combinaison d'un profil de libération pulsée, en rafales et prolongée.

8. Le stent de la revendication 7, dans lequel l'au moins un agent biologiquement actif est choisi parmi le paclitaxel, le docétaxel, l'estradiol, la dexaméthasone, le clobétasol, des donneurs d'oxyde nitrique, des superoxyde dismutases, des mimétiques de superoxyde dismutases, le 4-amino-2,2,6,6-tétraméthylpipéridine-1-oxyle (4-amino-TEMPO), le tacrolimus (FK-506), la rapamycine (sirolimus), des dérivés de rapamycine, la 40-O-(2-hydroxy)éthyl-rapamycine (évérolimus), la 40-O-(2-éthoxy)éthyl-rapamycine (biolimus), la 40-O-(3-hydroxy)propyl-rapamycine, la 40-O-[2-(2-hydroxy)éthoxy]éthyl-rapamycine, la 40-O-tétrazole-rapamycine, la 40-epi-(N1-tétrazolyl)-rapamycine (zotarolimus), le pimécrolimus, le mésylate d'imatinib, la midostaurine, des anticorps de capture de cellules progénitrices, des médicaments promoteurs de la guérison, des prodrogues de ceux-ci, des co-drogues de ceux-ci, et des combinaisons de ceux-ci.

9. Le stent de la revendication 7, dans lequel l'au moins un agent biologiquement actif a une libération prolongée pendant une période de jusqu'à 12 mois.

10. Le stent de la revendication 7, dans lequel l'au moins un agent biologiquement actif a une libération prolongée pendant une période de jusqu'à 9 mois.

11. Le stent de la revendication 7, dans lequel l'au moins un agent biologiquement actif a une libération prolongée pendant une période de jusqu'à 6 mois.

12. Le stent de la revendication 1, dans lequel la composition comprend en outre un ou plusieurs composants choisis parmi des restes biocompatibles, des restes anti-encrassement, des matériaux biologiquement avantageux, et des combinaisons de ceux-ci.

13. Le stent de la revendication 12, dans lequel :
l'un ou les plusieurs restes biocompatibles sont choisis parmi la phosphorylcholine, le poly(oxyde d'éthylène), le polypropylèneglycol, le polytétraméthylèneglycol, le poly(oxyde d'éthylène-co-oxyde de propylène), la caprolactone, la β-butyrolactone, la valérolactone, le glycolide, la poly(N-vinyl pyrrolidone), le poly(méthyl-acrylamide-acide propanesulfonique) et des sels de ceux-ci, le poly(styrène sulfonate), le dextrane sulfoné, les polyphosphazènes, les polyorthoésters, le poly(carbonate de tyrosine), un acide sialique, l'acide hyaluronique et des dérivés de celui-ci, des copolymères de polyéthylène glycol (PEG) avec acide hyaluronique ou dérivés de celui-ci, l'héparine, copolymères de PEG avec héparine, des copolymères greffés de poly(L-lysine) et PEG, et des dérivés et copolymères de ceux-ci ;
l'un ou les plusieurs restes anti-encrassement sont choisis parmi le polyéthylène glycol, le polypropylène glycol, les tensioactifs Pluronic™, le poly(2-hydroxyéthyl méthacrylate), le poly(alcool vinylique), les oxydes de polyalcène, la poly(n-propylméthacrylamide), la poly(N-vinyl-2-pyrrolidone), le polystyrène sulfoné, le dextran, le dextran sulfoné, la dextrine, l'acide hyaluronique, le hyaluronate de sodium, la phosphorylcholine, et des dérivés et copolymères de ceux-ci ; et
l'un ou les plusieurs matériaux biologiquement avantageux sont choisis parmi la fibrine, le fibrinogène, la cellulose et des dérivés de celle-ci, l'amidon, la pectine, le chitosane, l'élastine, la gélatine, l'alginate et des conjugués de celui-ci, le collagène et des conjugués de celui-ci, le hyaluronane et des dérivés de celui-ci, l'acide hyaluronique, le hyaluronate de sodium, les peptides auto-assemblés, et des dérivés et copolymères de ceux-ci.

14. Le stent de la revendication 1, comprenant en outre un revêtement comprenant un(des) polymère(s) biodégradable(s), un(des) polymère(s) biostable(s), ou une combinaison de ceux-ci.

15. Un stent tel que défini dans la revendication 1, pour l'utilisation dans le traitement ou la prévention d'une condition ou un trouble chez un patient choisi(e) dans le groupe constitué de l'athérosclérose, la thrombose, la resténose, des hémorragies, une dissection vasculaire, une perforation vasculaire, l'anévrisme vasculaire, la plaque vulnérable, l'occlusion totale chronique, le foramen ovale perméable, la claudication, la prolifération anastomotique pour des implants de veines et des implants artificiels, et les anastomoses artérioveineuses.

16. Le stent de la revendication 8, dans lequel l'au moins un agent biologiquement actif est choisi parmi le paclitaxel, le docétaxel, l'estradiol, la dexaméthasone, le clobétasol, le 4-amino-2,2,6,6-tétraméthylpipéridine-1-oxyle (4-amino-TEMPO), le tacrolimus (FK-506), la rapamycine (sirolimus), la 40-O-(2-hydroxy)éthyl-rapamycine (évérolimus), la 40-O-(2-éthoxy)éthyl-rapamycine (biolimus), la 40-O-(3-hydroxy)propyl-rapamycine, la 40-O-[2-(2-hydroxy)éthoxy]éthyl-rapamycine, la 40-O-tétrazole-rapamycine, la 40-epi-(N1-tétrazolyl)-rapamycine (zotarolimus), le pimécrolimus, le mésylate d'imatinib, la midostaurine, et des combinaisons de ceux-ci.
